# EUROPÄISCHE PATENTSCHRIFT

(11) **EP 1 777 246 B1**
(45) Veröffentlichungstag und Bekanntmachung des Hinweises auf die Patenterteilung: **06.06.2012**
(21) Anmeldenummer: 06021298.2
(22) Anmeldetag: 11.10.2006
(51) Int. Cl.: C08G 69/34, C08G 69/40, C08G 69/44, C08G 69/48, C07C 233/36, C07C 233/38, C07C 237/06, C07C 237/10, C07C 275/40, C09D 7/00

(54) **Amidhaltige Polymere zur Rheologiesteuerung**
Polymers containing amide groups for controlling rheology
Polymères contenant des groupes amides pour réguler la rhéologie

(30) Priorität: 12.10.2005 DE 102005049301
(43) Veröffentlichungstag der Anmeldung: 25.04.2007
(73) Patentinhaber: BYK-Chemie GmbH, 46483 Wesel (DE)
(72) Erfinder: Haubennestel, Karlheinz, 46487 Wesel (DE); Mössmer, Stefan, Dr., 46487 Wesel (DE); Orth, Ulrich, Dr., 46485 Wesel (DE); Leutfeld, Daniela, 46485 Wesel (DE)
(74) Vertreter: Leifert & Steffan

(56) Entgegenhaltungen:
- EP-A1- 0 936 254
- EP-A2- 0 277 420
- EP-A2- 0 432 943
- EP-A2- 0 528 363
- EP-A2- 0 692 509
- DE-A1- 1 495 941
- US-A- 4 107 061
- US-B1- 6 211 249

## Beschreibung

Die Erfindung betrifft amidhaltige Polymere zur Rheologiesteuerung von flüssigen Polymersystemen wie beispielsweise lösungsmittelhaltigen, lösungsmittelfreien und wässrigen Lacken, PVC-Plastisolen, Beschichtungen auf Epoxidbasis und ungesättigten Polyesterharzen.

Um die Rheologie von flüssigen Systemen zu steuern, werden vielfach Kieselsäuren, hydriertes Rizinusöl oder organisch modifizierte Bentonite, wie zum Beispiel in US 4,208,218, US 4,410,364 und US 4,412,018 beschrieben, eingesetzt. Des Weiteren kommen vielfach Polyamidwachse zum Einsatz. Auf dem Gebiet der Polyamide und Polyamidester existieren zahlreiche Patente, wie beispielsweise die EP 0 692 509, EP 0277420, EP 0528363 EP 0239419, US 5,510,452 und US 5,349,011. Es werden aber auch Kombinationen von modifizierten Bentoniten mit Polyamiden, wie in der EP 0509202 und EP 0826750 beschrieben, eingesetzt.

Nachteilig bei diesen Stoffen ist, dass sie meist trockene Feststoffe oder Pasten darstellen, die mittels Lösungsmitteln und Aufwendung von Scherkräften zu einem Halbfabrikat aufgeschlossen, beziehungsweise durch gezielte Temperatursteuerung in beispielsweise flüssige Beschichtungssysteme eingebracht werden müssen. Werden die notwendigen Temperaturen nicht eingehalten, treten im fertigen Beschichtungssytem Kristallite auf, die zu Fehlern in der Beschichtung führen. Der generelle Nachteil dieser Stoffe ist, dass sie zu Trübungen und Schleierbildungen (Haze) in klaren, transparenten Beschichtungen führen. Außerdem ist der Umgang mit trockenen Produkten, die Stäube bei der Verarbeitung verursachen, nicht gewünscht.

Die Polyamidester sind zwar häufig flüssig, aber deutlich weniger wirksam, als die von Natur aus festen Stoffe.

Andere Lösungen zur Rheologiesteuerung wurden in der EP 0 198 519 dargestellt. Hier wird ein Isocyanat mit einem Amin in Gegenwart von Bindemitteln zu einem Harnstoff umgesetzt, der in feinst disperser Form nadelförmige Kristalle bildet. Diese so modifizierten Bindemittel werden als rheologiesteuernde und ablaufverhindernde Bindemittel angeboten, und als "sag control agents" bezeichnet.

Der Nachteil dieser Produkte liegt darin, dass sie immer an die Bindemittel gebunden sind, in denen sie hergestellt wurden und somit eine nachträgliche universelle Korrektur von fertigen Systemen nicht möglich ist.

In der EP 0 006 252 wird ein Verfahren zur Herstellung eines Thixotropiemittels beschrieben, das einige der oben genannten Nachteile ausräumt, in dem es Harnstoffurethane bereitstellt, die in aprotischen Lösungsmitteln in Gegenwart von LiCl durch Umsetzung von Isocyanataddukten mit Polyaminen hergestellt werden. Der Nachteil der so hergestellten Produkte liegt in der durch das Herstellverfahren bedingten undefinierten Struktur der Harnstoffurethane. Bei besagtem Verfahren werden Diisocyanate und Monoalkohole in gleichen Molmengen eingesetzt. Dabei entstehen sowohl NCO-funktionelle Monoaddukte, als auch nicht-NCO-funktionelle Diaddukte. Außerdem bleibt ein gewisser Anteil an monomerem Diisocyanat nicht umgesetzt. Die Anteile der verschiedenen Produkte schwanken, je nach Zugänglichkeit der NCO-Gruppe und der angewandten Reaktionsführung, wie Temperatur und Zeit. Alle so hergestellten Addukte enthalten jedoch größere Mengen an nicht umgesetztem Diisocyanat, welches bei der weiteren Umsetzung mit Polyaminen zu unkontrollierter Kettenverlängerung des Moleküls führt. Diese Produkte neigen dann zu Ausfällungserscheinungen oder vorzeitiger Gelierung und demzufolge zur Bildung von so genannten "Stippen" ("seeds") im Bindemittel. In der US 6,420,466 werden diese Nachteile durch Entfernen des überschüssigen Isocyanates umgangen. Die dort beschriebenen Produkte haben jedoch den Nachteil, dass sie nur in hochpolaren Lösemitteln wie z.B. N-Methylpyrrolidon (NMP) unter Zuhilfenahme von Alkalisalzen stabile Lösungen liefern.

Der vorliegenden Erfindung liegt daher die Aufgabe zugrunde, Produkte bereit zu stellen, welche zur Rheologiesteuerung flüssiger Polymersysteme geeignet sind, Polymere einer definierteren Struktur umfassen und so ein besseres Wirkungsprofil und eine bessere Reproduzierbarkeit der Rheologiesteuerung gewährleisten, und die insbesondere die oben geschilderten Nachteile der Additive des Stands der Technik vermeiden.

Überraschenderweise wurde gefunden, dass diese Aufgaben durch Bereitstellung amidhaltiger Polymere der allgemeinen Formel (I)

A-X-CO-(CH₂)₂-NR¹-R²-[Y-R³-Y-R⁴]ₐ-B (I)

sowie deren Salze mit Carbonsäuren, Phosphorsäureestern und Sulfonsäuren,
wobei
A für R⁵ oder R⁶-Y-[R⁴-Y-R³-Y]_{b}-R²-NR¹-(CH₂)₂-CO-X-R⁷ und
B für Y-R⁶ oder NR¹-(CH₂)₂-CO-X-R⁵ steht, und wobei
R¹ für H, (CH₂)₂-CO-X-R⁵ und/oder CONH-R' mit R' = R⁸ oder -C₆H₃(CH₃)-NHCOO-R⁸ steht,
R², R³, R⁴ und R⁷ unabhängig voneinander für einen (C₁-C₄₀)Alkylen-, (C₃-C₄₀)Alkenylen-, (C₅-C₄₀)Cycloalkylen-, Arylen-, (C₇-C₄₀)Aralkylen- oder Polyoxyalkylen-Rest oder für einen Polyester-Rest stehen,
R⁵ für H, einen (C₁-C₂₂)Alkyl-, Aryl-, (C₇-C₁₂)Aralkyl-, (C₅-C₁₂)Cycloalkyl-, Hydroxyalkyl-, oder N,N'-Dialkylamino-Rest, einen Hydroxy-, (C₁-C₂₂)Alkoxy-, (C₅-C₁₂)Cycloalkoxy-, oder (C₇-C₁₂)Aralkoxy-Polyoxyalkylen-Rest, oder einen (C₁-C₂₂)Alkanol-, (C₅-C₁₂)Cycloalkanol-, (C₇-C₁₂)Aralkanol-gestarteten oder einen (C₁-C₂₂)Alkoxy-, (C₅-C₁₂)Cycloalkoxy-, oder (C₇-C₁₂)Aralkoxy-Polyoxyalkylen-gestarteten Polyester steht,
R⁶ für einen (C₁-C₃₀)Alkyl-, (C₃-C₂₂)Alkenyl-, Hydroxyalkyl-, (C₄-C₁₃)Cycloalkyl-, Aryl- oder (C₇-C₁₂)Aralkyl-Rest steht,
R⁸ für einen (C₁-C₂₂)Alkyl-, Aryl-, (C₇-C₁₂)Aralkyl-, oder (C₅-C₁₂)Cycloalkyl-Rest, einen (C₁-C₂₂)Alkoxy-, (C₅-C₁₂)Cycloalkoxy-, oder (C₇-C₁₂)Aralkoxy-Polyoxyalkylen-Rest, einen (C₁-C₂₂)Alkanol-, (C₅-C₁₂)Cycloalkanol-, oder (C₇-C₁₂)Aralkanol-gestarteten oder einen (C₁-C₂₂)Alkoxy-, (C₆-C₁₂)Cycloalkoxy-, oder (C₇-C₁₂)Aralkoxy-Polyoxyalkylen-gestarteten Polyester steht,
X für gleiche oder verschiedene Reste O, NH oder NR⁹ steht,
R⁹ für einen (C₁-C₂₂)Alkyl-, Aryl-, (C₇-C₁₂)Aralkyl-, Hydroxyalkyl-, (C₅-C₁₂)CycloalkylRest steht,
Y für eine oder mehrere der folgenden Gruppen COO, OCO, NHCO, CONH, NHCOO, OOCNH, NHCONH steht und
a und b unabhängig voneinander für eine Zahl von 1 bis 19 stehen, gelöst werden.

Die Definitionen der Reste A, B, R¹, R², R³, R⁴, R⁵, R⁶, R⁷, R⁸, R⁹, R', X und Y sowie der Indices a und b entsprechen im Rahmen dieser Erfindung unabhängig von den Verbindungen, in welchen diese aufgeführt sind, den obigen Definitionen. Bevorzugte Ausführungen dieser Reste finden sich in den jeweiligen Unterabschnitten.

Im Falle, dass einer oder mehrere der Reste R², R³, R⁴, R⁵, R⁷ und/oder R⁸ einen Polyoxyalkylen-Anteil enthalten, sind diese Reste unabhängig von den Verbindungen, in welchen diese aufgeführt sind, vorzugsweise aus Ethylenoxid, Propylenoxid und/oder Butylenoxid-Einheiten, statistisch oder blockweise angeordnet, aufgebaut, und gegebenenfalls ist einer oder sind mehrere dieser Einheiten durch Styrol-Einheiten substituiert. Besonders bevorzugt sind Ethylenoxid- und Propylenoxid-Reste.

Im Falle, dass einer oder mehrere der Reste R², R³, R⁴, R⁵, R⁷ und/oder R⁸ einen Polyester-Rest umfassen, sind diese Reste unabhängig von den Verbindungen, in welchen diese aufgeführt sind, vorzugsweise auf der Basis von ein oder mehreren (C₁-C₁₈)-Hydroxycarbonsäuren oder ein oder mehreren Lactonen, wie β-Propiolacton, δ-Valerolacton, ε-Caprolacton und (C₁-C₆)Alkylsubstituiertem ε-Caprolacton, aufgebaut.

Die Reste R¹ stehen für H, (CH₂)₂-CO-X-R⁵ und/oder CONH-R' mit R = R⁸ oder -C₆H₃(CH₃)-NHCOO-R⁸, wie beispielsweise CONH-C₁₈H₃₇ und/oder CONH-C₆H₃(CH₃)-NHCOOC₄H₉.

Die Reste R² und R⁴ stehen unabhängig voneinander vorzugsweise für einen (C₂-C₁₈)Alkylen-, (C₇-C₁₅)Aralkylen-Rest, besonders bevorzugt für einen (C₂-C₁₂)Alkylen-, (C₇-C₁₂)Aralkylen-Rest, ganz besonders bevorzugt für einen (C₂-C₈)Alkylen-, (C₇-C₉)Aralkylen-Rest, wie beispielsweise einen Hexamethylen-, Octamethylen- oder m-Xylylen-Rest. Bevorzugt sind die Reste R² und R⁴ identisch.

R³ steht für einen (C₂-C₄₀)Alkylen-, (C₃-C₄₀)Alkenylen-, (C₅-C₄₀)Cycloalkylen-, Arylen- oder (C₇-C₄₀)Aralkylen-Rest, bevorzugt für einen (C₃₀-C₄₀)Alkylen-, (C₃₀-C₄₀)Alkenylen-, (C₃₀-C₄₀)Cycloalkylen-, Arylen-, oder (C₃₀-C₄₀)Aralkylen-Rest, wie beispielsweise den Rest zwischen den beiden Carbonsäuregruppen der Dimersäure. Besonders bevorzugt steht R³ für einen C₃₄-Rest.

R⁵ steht vorzugsweise für einen (C₁-C₂₂)Alkyl-, einen Hydroxy- oder einen Alkoxypolyoxyalkylenrest.

R⁶ steht vorzugsweise für einen (C₁-C₃₀)Alkyl- oder einen (C₃-C₂₂)Alkenyl-Rest, besonders bevorzugt einen (C₁₂-C₃₀)Alkyl- oder einen (C₁₂-C₂₂)Alkenyl-Rest und ganz besonders bevorzugt einen (C₁₂-C₂₀)Alkyl- oder einen (C₁₂-C₂₀)Alkenyl-Rest, wie beispielsweise einen C₁₇-Alkyl- oder einen C₁₇-Alkenyl-Rest.

R⁷ steht vorzugsweise für einen (C₁-C₁₈)Alkylen- oder einen Polyoxyalkylenrest und R⁸ für einen (C₁-C₂₂)Alkyl-Rest. X steht vorzugsweise für gleiche oder verschiedene Reste O oder NH, und Y vorzugsweise für eine oder mehrere der Gruppen NHCO und CONH.

Die Indices a und b stehen unabhängig voneinander für eine Zahl von 1 bis 19, besonders bevorzugt von 2 bis 7, und sind vorzugsweise identisch.

Besonders bevorzugte Ausführungsformen der Erfindung betreffen Verbindungen der allgemeinen Formel (I) mit A = R⁵ und B = NR¹-(CH₂)₂-CO-X-R⁵, woraus sich die allgemeine Formel (II) ergibt:

R⁵-X-CO-(CH₂)₂-NR¹-R²-[Y-R³-Y-R⁴]ₐ-NR¹-(CH₂)₂-CO-X-R⁵ (II),

Verbindungen der allgemeinen Formel (I) mit A = R⁵ und B = Y-R⁶, woraus sich die allgemeine Formel (III) ergibt:

R⁵-X-CO-(CH₂)₂-NR¹-R²-[Y-R³-Y-R⁴]ₐ-Y-R⁶ (III),

und Verbindungen der allgemeinen Formel (I) mit
A = R⁶-Y-[R⁴-Y-R³-Y]ₐ-R²-NR¹-(CH₂)₂-CO-X-R⁷ und B = Y-R⁶, woraus sich die allgemeine Formel (IV) ergibt:

R⁶-Y-[R⁴-Y-R³-Y]ₐ-R²-NR¹-(CH₂)₂-CO-X-R⁷-X-CO-(CH₂)₂-NR¹-R²-[Y-R³-Y-R⁴]ₐ-Y-R⁶ (IV)

Die vorliegende Erfindung betrifft neben den amidhaltigen Polymeren auch ein Verfahren zur Herstellung amidhaltiger Polymere der allgemeinen Formel (I)

A-X-CO-(CH₂)₂-NR¹-R²-[Y-R³-Y-R⁴]ₐ-B (I)

sowie deren Salze mit Carbonsäuren, Phosphorsäureestern und Sulfonsäuren,
wobei
A für R⁵ oder R⁶-Y-[R⁴-Y-R³-Y]_{b}-R²-NR¹-(CH₂)₂-CO-X-R⁷ und
B für Y-R⁶ oder NR¹-(CH₂)₂-CO-X-R⁵ steht, und wobei
R¹ für H, (CH₂)₂-CO-X-R⁵ und/oder CONH-R' mit R' = R⁸ oder -C₆H₃(CH₃)-NHCOO-R⁸ steht,
R², R³, R⁴ und R⁷ unabhängig voneinander für einen (C₁-C₄₀)Alkylen-, (C₃-C₄₀)Alkenylen-, (C₅-C₄₀)Cycloalkylen-, Arylen-, (C₇-C₄₀)Aralkylen- oder Polyoxyalkylen-Rest oder für einen Polyester-Rest stehen,
R⁵ für H, einen (C₁-C₂₂)Alkyl-, Aryl-, (C₇-C₁₂)Aralkyl-, (C₅-C₁₂)Cycloalkyl-, Hydroxyalkyl-, oder N,N'-Dialkylamino-Rest, einen Hydroxy-, (C₁-C₂₂)Alkoxy-, (C₅-C₁₂)Cycloalkoxy-, oder (C₇-C₁₂)Aralkoxy-Polyoxyalkylen-Rest, oder einen (C₁-C₂₂)Alkanol-, (C₅-C₁₂)Cycloalkanol-, (C₇-C₁₂)Aralkanol-gestarteten oder einen (C₁-C₂₂)Alkoxy-, (C₅-C₁₂)Cycloalkoxy-, oder (C₇-C₁₂)Aralkoxy-Polyoxyalkylen-gestarteten Polyester steht,
R⁶ für einen (C₁-C₃₀)Alkyl-, (C₃-C₂₂)Alkenyl-, Hydroxyalkyl-, (C₄-C₁₃)Cycloalkyl-, Aryl- oder (C₇-C₁₂)Aralkyl-Rest steht,
R⁸ für einen (C₁-C₂₂)Alkyl-, Aryl-, (C₇-C₁₂)Aralkyl-, oder (C₅-C₁₂)Cycloalkyl-Rest, einen (C₁-C₂₂)Alkoxy-, (C₅-C₁₂)Cycloalkoxy-, oder (C₇-C₁₂)Aralkoxy-Polyoxyalkylen-Rest, einen (C₁-C₂₂)Alkanol-, (C₅-C₁₂)Cycloalkanol-, oder (C₇-C₁₂)Aralkanol-gestarteten oder einen (C₁-C₂₂)Alkoxy-, (C₆-C₁₂)Cycloalkoxy-, oder (C₇-C₁₂)Aralkoxy-Polyoxyalkylen-gestarteten Polyester steht,
X für gleiche oder verschiedene Reste O, NH oder NR⁹ steht,
R⁹ für einen (C₁-C₂₂)Alkyl-, Aryl-, (C₇-C₁₂)Aralkyl-, Hydroxyalkyl-, (C₅-C₁₂)CycloalkylRest steht,
Y für eine oder mehrere der folgenden Gruppen COO, OCO, NHCO, CONH, NHCOO, OOCNH, NHCONH steht und
a und b unabhängig voneinander für eine Zahl von 1 bis 19 stehen,
worin,
(A) eine oder mehrere Verbindungen der allgemeinen Formeln (V) und (VIII)

   H₂N-R²-[Y-R³-Y-R⁴]ₐ-NH₂ (V)

   H₂N-R²-[Y-R³-Y-R⁴]ₐ-YR⁶ (VIII)

   mit einer oder mehreren Verbindungen der allgemeinen Formeln (VI) und (IX)

   R⁵-X-CO-CH=CH₂ (VI)

   H₂C=HC-CO-X-R⁷-X-CO-CH=CH₂ (IX)

   unter Bildung von Verbindungen mit R¹ = Wasserstoff zur Reaktion gebracht werden, wobei
   auf 1 Mol NH₂-Gruppen in den Verbindungen der allgemeinen Formeln (V) und (VIII) 0,8 bis 1,2 Mol CH=CH₂-Gruppen in den Verbindungen der Formeln (VI) und (IX) eingesetzt werden, und
(B) für den Fall, dass R¹ ganz oder teilweise für (CH₂)₂-CO-X-R⁵ und/oder CONH-R' steht, die Verbindungen aus Schritt (A) mit
   einer oder mehreren Verbindungen der allgemeinen Formeln (VI) und (VII)

   R⁵-X-CO-CH=CH₂ (VI)

   R'-NCO (VII)

   zur Reaktion gebracht werden, wobei
   auf 1 Mol NR¹-Gruppen in den Verbindungen aus Schritt (A) bis zu 1,2 Mol an Verbindungen der allgemeinen Formel (VI) und/oder (VII) eingesetzt werden, und
(C) für den Fall, dass es sich bei den Verbindungen der allgemeinen Formel (I) um Salze von Carbonsäuren, Phosphorsäureestern und Sulfonsäuren handelt, eine Umsetzung der Verbindungen aus Schritt (A) oder (B) mit Carbonsäuren, Phosphorsäureestern und Sulfonsäuren erfolgt.
   In einer bevorzugten Ausführungsform werden

(A) eine oder mehrere Verbindungen der allgemeinen Formel (V)

   H₂N-R²-[Y-R³-Y-R⁴]ₐ-NH₂ (V)

   mit einer oder mehreren Verbindungen der allgemeinen Formel (VI)

   R⁵-X-CO-CH=CH₂ (VI)

   unter Bildung von Verbindungen mit R¹ = Wasserstoff zur Reaktion gebracht,
   wobei
   auf 1 Mol NH₂-Gruppen in den Verbindungen der allgemeinen Formel (V) 0,8 bis 1,2 Mol CH=CH₂-Gruppen in den Verbindungen der Formel (VI) eingesetzt werden, und
(B) für den Fall, dass R¹ ganz oder teilweise für (CH₂)₂-CO-X-R⁵ und/oder CONH-R' steht, die Verbindungen aus Schritt (A) mit
   einer oder mehreren Verbindungen der allgemeinen Formeln (VI) und (VII)

   R⁵-X-CO-CH=CH₂ (VI)

   R'-NCO (VII)

   zur Reaktion gebracht werden, wobei
   auf 1 Mol NR¹-Gruppen in den Verbindungen aus Schritt (A) bis zu 1,2 Mol an Verbindungen der allgemeinen Formel (VI) und/oder (VII) eingesetzt werden, und
(C) für den Fall, dass es sich bei den Verbindungen der allgemeinen Formel (I) um Salze von Carbonsäuren, Phosphorsäureestern und Sulfonsäuren handelt, eine Umsetzung der Verbindungen aus Schritt (A) oder (B) mit Carbonsäuren, Phosphorsäureestern und Sulfonsäuren erfolgt.
   In einer weiteren bevorzugten Ausführungsform werden

(A) eine oder mehrere Verbindungen der allgemeinen Formel (VIII)

   H₂N-R²-[Y-R³-Y-R⁴]ₐ-YR⁶ (VIII)

   mit einer oder mehreren Verbindungen der allgemeinen Formel (VI)

   R⁵-X-CO-CH=CH₂ (VI)

   unter Bildung von Verbindungen mit R¹ = Wasserstoff zur Reaktion gebracht,
   wobei
   auf 1 Mol NH₂-Gruppen in den Verbindungen der allgemeinen Formel (VIII) 0,8 bis 1,2 Mol CH=CH₂-Gruppen in den Verbindungen der Formel (VI) eingesetzt werden, und
(B) für den Fall, dass R¹ ganz oder teilweise für (CH₂)₂-CO-X-R⁵ und/oder CONH-R' steht, die Verbindungen aus Schritt (A) mit
   einer oder mehreren Verbindungen der allgemeinen Formeln (VI) und (VII)

   R⁵-X-CO-CH=CH₂ (VI)

   R'-NCO (VII)

   zur Reaktion gebracht werden, wobei
   auf 1 Mol NR¹-Gruppen in den Verbindungen aus Schritt (A) bis zu 1,2 Mol an Verbindungen der allgemeinen Formel (VI) und/oder (VII) eingesetzt werden, und
(C) für den Fall, dass es sich bei den Verbindungen der allgemeinen Formel (I) um Salze von Carbonsäuren, Phosphorsäureestern und Sulfonsäuren handelt, eine Umsetzung der Verbindungen aus Schritt (A) oder (B) mit Carbonsäuren, Phosphorsäureestern und Sulfonsäuren erfolgt.
   In einer weiteren bevorzugten Ausführungsform werden

(A) eine oder mehrere Verbindungen der allgemeinen Formel (VIII)

   H₂N-R²-[Y-R³-Y-R⁴]ₐ-YR⁶ (VIII)

   mit einer oder mehreren Verbindungen der allgemeinen Formel (IX)

   H₂C=HC-CO-X-R⁷-X-CO-CH=CH₂ (IX)

   unter Bildung von Verbindungen mit R¹ = Wasserstoff zur Reaktion gebracht,
   wobei
   auf 1 Mol NH₂-Gruppen in den Verbindungen der allgemeinen Formel (VIII) 0,8 bis 1,2 Mol CH=CH₂-Gruppen in den Verbindungen der Formel (IX) eingesetzt werden, und
(B) für den Fall, dass R¹ ganz oder teilweise für (CH₂)₂-CO-X-R⁵ und/oder CONH-R' steht, die Verbindungen aus Schritt (A) mit
   einer oder mehreren Verbindungen der allgemeinen Formeln (VI) und (VII)

   R⁵-X-CO-CH=CH₂ (VI)

   R'-NCO (VII)

   zur Reaktion gebracht werden, wobei
   auf 1 Mol NR¹-Gruppen in den Verbindungen aus Schritt (A) bis zu 1,2 Mol an Verbindungen der allgemeinen Formel (VI) und/oder (VII) eingesetzt werden, und
(C) für den Fall, dass es sich bei den Verbindungen der allgemeinen Formel (I) um Salze von Carbonsäuren, Phosphorsäureestern und Sulfonsäuren handelt, eine Umsetzung der Verbindungen aus Schritt (A) oder (B) mit Carbonsäuren, Phosphorsäureestern und Sulfonsäuren erfolgt.

In den genannten bevorzugten Ausführungsformen des erfindungsgemäßen Verfahrens kann die in Schritt (B) angegebene Menge der Verbindungen der allgemeinen Formel (VI) anstatt in Schritt (B), bereits in Schritt (A) zusätzlich zu der in Schritt (A) angegebenen Menge der Verbindungen der allgemeinen Formel (VI) eingesetzt werden.

Die Reaktion in Schritt (A) wird vorzugsweise bei einer Temperatur von 60 bis 100 °C, besonders bevorzugt bei einer Temperatur von 70 bis 90 °C durchgeführt.

Die Reaktion in Schritt (B), wird im Falle der Umsetzung mit Verbindungen der allgemeinen Formel (VII) vorzugsweise bei einer Temperatur von 50 bis 100 °C, besonders bevorzugt 60 bis 80 °C und im Falle der Umsetzung mit einer Verbindung der allgemeinen Formel (VI) bei einer Temperatur von vorzugsweise 60 bis 100 °C, besonders bevorzugt 70 bis 90 °C durchgeführt.

Im folgenden wird das erfindungsgemäße Verfahren an konkreten Beispielen erläutert.

Verbindungen der Formel (II) mit R¹ = -H, -(CH₂)₂-CO-X-R⁵, -CONH-R' mit R'= R⁸ oder -C₆H₃(CH₃)-NHCOO-R⁸ lassen sich beispielsweise herstellen, indem eine oder mehrere Verbindungen der allgemeinen Formel (V):

H₂N-R²-[Y-R³-Y-R⁴]ₐ-NH₂ (V)

wobei R², R³ und R⁴ unabhängig voneinander für einen (C₂-C₄₀)Alkylen-, (C₃-C₄₀)Alkenylen-, (C₅-C₄₀)Cycloalkylen, Arylen-, (C₇-C₄₀)Aralkylen- oder Polyoxyalkylen-Rest stehen,
Y für eine oder mehrere der folgenden Gruppen COO, OCO, NHCO, CONH, NHCOO, OOCNH, NHCONH steht, und
a für eine Zahl von 1 bis 19 steht,
mit einer oder mehreren Verbindungen der allgemeinen Formel (VI):

R⁵-X-CO-CH=CH₂ (VI)

wobei R⁵ für H, einen (C₁-C₂₂)Alkyl-, Aryl-, (C₇-C₁₂)Aralkyl-, (C₅-C₁₂)Cycloalkyl-, Hydroxyalkyl-, oder N,N'-Dialkylaminorest, einen Hydroxy-, (C₁-C₂₂)Alkoxy-, (C₅-C₁₂)Cycloalkoxy-, oder (C₇-C₁₂)Aralkoxypolyoxyalkylenrest, wobei es sich bei dem Oxyalkylenrest um Ethylen-, Propylen- oder Butylenoxid, einschließlich deren Mischungen, handelt und Teile des Oxyalkylenrestes durch Styroloxid substituiert sein können oder einen (C₁-C₂₂)Alkanol-, (C₅-C₁₂)Cycloalkanol-, (C₇-C₁₂)Aralkanolgestarteten oder einen (C₁-C₂₂)Alkoxy-, (C₅-C₁₂)Cycloalkoxy-, oder (C₇-C₁₂)Aralkoxypolyoxyalkylengestarteten Polyester auf Basis von z.B. (C₁-C₁₈)Hydroxycarbonsäuren oder Lactonen wie z.B. β-Propiolacton, δ-Valerolacton, ε-Caprolacton oder (C₁-C₆)Alkylsubstituiertem ε-Caprolacton, einschließlich Mischungen davon, steht,
X für O, NH oder NR⁹ steht, und
R⁹ für (C₁-C₂₂)Alkyl-, Aryl-, (C₇-C₁₂)Aralkyl-, Hydroxyalkyl-, (C₅-C₁₂)Cycloalkylrest steht, umgesetzt werden
und anschließend das Produkt gegebenenfalls mit Verbindungen der allgemeinen Formel (VII):

R'-NCO (VII)

wobei R' für R⁸ oder -C₆H₃(CH₃)-NHCOO-R⁸ steht und
R⁸ für einen (C₁-C₂₂)Alkyl-, Aryl-, (C₇-C₁₂)Aralkyl-, oder (C₅-C₁₂)Cycloalkylrest, einen (C₁-C₂₂)Alkoxy-, (C₅-C₁₂)Cycloalkoxy-, oder (C₇-C₁₂)Aralkoxypolyoxyalkylenrest,
wobei es sich bei dem Oxyalkylenrest um Ethylen-, Propylen- oder Butylenoxid, einschließlich deren Mischungen, handelt und Teile des Oxyalkylenrestes gegebenenfalls durch Styroloxid substituiert sind, einen (C₁-C₂₂)Alkanol-, (C₅-C₁₂)Cycloalkanol-, oder (C₇-C₁₂)Aralkanolgestarteten oder einen (C₁-C₂₂)Alkoxy-, (C₆-C₁₂)Cycloalkoxy-, oder (C₇-C₁₂)Aralkoxypolyoxyalkylengestarteten Polyester auf Basis von z.B. (C₁-C₁₈)Hydroxycarbonsäuren oder Lactonen wie z.B. β-Propiolacton, δ-Valerolacton, ε-Caprolacton oder (C₁-C₆)Alkylsubstituiertem ε-Caprolacton, einschließlich Mischungen davon, steht,
umgesetzt wird.

Die erfindungsgemäßen Zwischenverbindungen (V) sind beispielsweise dadurch erhältlich, dass man Polycarbonsäuren, bevorzugt Dicarbonsäuren, und/oder Dicarbonsäureanhydride mit Diaminen umsetzt, wobei das Verhältnis Diamin zu Polycarbonsäure 2 zu 1 bis 20 zu 19, besonders bevorzugt 3 zu 2 bis 8 zu 7 beträgt.

Bei den Diaminen handelt es sich vorzugsweise um aliphatische und araliphatische primäre Diamine, wie z. B. Ethylendiamin, Neopentandiamin, 1,2- und 1,3-Propandiamin, 1,4-Butandiamin, 1,5-Pentandiamin, 1,6-Hexamethylendiamin (auch als Lösung in Wasser), 1,8-Octamethylendiamin, 1,12-Dodecamethylendiamin, Cyclohexyldiamin, 4,4'-Diaminodicyclohexylmethan, 3,3'-Dimethyl-4,4'-Diaminodicyclohexylmethan, Isophorondiamin, 4,7-Dioxadecan-1,10-diamin, 4,11-Dioxatetradecan-1,14-diamin, 4,7,10-Trioxadecan-1,13-diamin, Polyoxyalkylendiamine, welche Ethylenoxid- und/oder Propylenoxid-Gruppen, statistisch oder blockweise angeordnet, enthalten, ein zahlenmittleres Molekulargewicht von 148 bis 4000 g/mol besitzen und beispielsweise als Jeffamine^{®} D und Jeffamine^{®} ED von Huntsman erhältlich sind, Polytetrahydrofurandiamine sowie para- und meta-Xylylendiamin. Bevorzugt werden 1,6-Hexamethylendiamin, 1,8-Octamethylendiamin und meta-Xylylendiamin eingesetzt. Ebenfalls können Amine vom Typus H₂N-R-NR-R-NH₂ verwendet werden, wobei R unabhängig für (C₁-C₁₈)Alkyl oder (C₁-C₄)Alkoxy steht. Ein Beispiel hierfür ist N,N'-Bis-(3-aminopropyl)methylamin. Es können aber auch Dihydrazide wie z.B. Oxalsäuredihydrazid, Bernsteinsäuredihydrazid oder Adipinsäuredihydrazid eingesetzt werden. Der Einsatz von Mischungen der Diamine, auch mit den Dihydraziden, ist ebenfalls möglich. Die Diamine können auch als Carbonatverbindungen eingesetzt werden, die bei der Kondensationsreaktion mit den Polycarbonsäuren unter Wasserabscheidung und CO₂-Abspaltung zu den erfindungsgemäß bevorzugten Amidgruppierungen reagieren.

Bei den Polycarbonsäuren handelt es sich vorzugsweise um aliphatische, cycloaliphatische oder aromatische, lineare oder verzweigte, gesättigte oder ungesättigte Dicarbonsäuren mit wenigstens 2, besonders bevorzugt 3 bis 40 C-Atomen. Beispiele solcher Polycarbonsäuren sind Adipinsäure, Oxalsäure, Malonsäure, Bernsteinsäure, Glutarsäure, Pimelinsäure, Korksäure, Sebacinsäure, Azelainsäure, Undecandisäure, 1,11- Undecandicarbonsäure, Dodecandisäure, Hexadecandisäure, Dokosandisäure Maleinsäure, Fumarsäure, Terephthalsäure oder Isophthalsäure, alleine oder in Mischungen eingesetzt. Säureanhydride wie Maleinsäureanhydrid, Glutarsäureanhydrid, Phthalsäureanhydrid und Bernsteinsäureanhydrid, welche gegebenenfalls mit Alkyl- oder Alkylengruppen modifiziert sind wie z.B. Dodecenylbernsteinsäureanhydrid, sind ebenfalls in der Erfindung eingeschlossen. Polymere Polycarbonsäuren wie z.B. die Dicarbonsäure des Polybutadiens können ebenso verwendet werden wie hydroxyfunktionelle Polycarbonsäuren wie z.B. Weinsäure, Zitronensäure und Hydroxyphthalsäure. Oxydicarbonsäuren wie 3,6,9-Trioxyundecandisäure und Polyglykoldisäure sind ebenfalls mit eingeschlossen. Dimerisierte Fettsäuren, dem Fachmann als Dimersäuren bekannt, mit einer Kohlenstofflänge von 36 C-Atomen sind ganz besonders bevorzugt. Diese Dimersäuren können sowohl einen geringen Monomergehalt (üblicherweise < 8 Gewichtsprozent), als auch einen Anteil von nicht mehr als 25 Gewichtsprozent an Trimersäure aufweisen.

Die Polycarbonsäuren können teilweise durch Diisocyanate und die Diamine teilweise durch Diole ersetzt werden, wobei dann Ester-, Urethan- und/oder Harnstoffgruppen neben den bevorzugten Amidgruppierungen in den Verbindungen der allgemeinen Formel (V) vorliegen können.

Bei den Diolen handelt es sich, alleine oder in Mischungen, vorzugsweise um Polyoxyalkylendiole, Polyoxyalkenyldiole, die gegebenenfalls mit (C₁-C₄)Alkyl- und/oder Alkoxygruppen modifiziert sind, um Polyesterdiole, gemischte Polyesterpolyoxyalkylendiole, Polylactondiole, gemischte Polyoxyalkylenpolylactondiole, Polycarbonatdiole, Polyolefindiole, Polyacrylatdiole, alkoxylierte Bisphenol A Diole, um Diole vom Typ α-ω-Dihydroxyalkylensiloxane als auch deren alkoxylierte Verbindungen mit einem mittleren Molekulargewicht Mₙ von 250 bis 5000 g/mol.

Als Diisocyanate können vorzugsweise aliphatische, cycloaliphatische und aromatische Diisocyanate, oder deren Mischungen eingesetzt werden. Beispiele solcher Diisocyanate sind 1,4-Tetramethylendiisocyanat, 1,6-Hexamethylendiisocyanat, 2,2,4-Trimethyl-1,6-Hexamethylendiisocyanat, 1,10-Decamethylendiisocyanat, 1,4,-Cyclohexylendiisocyanat, p-Phenylen-Diisocyanat, m-Phenylen-Diisocyanat, 2,6-Toluylendiisocyanat, 2,4-Toluylendiisocyanat und deren Mischungen, p- und m-Xylylendiisocyanat, 1,5-Naphthylendiisocyanat, Isophorondiisocyanat, 4-4',-Diisocyanatodicyclohexylmethan, 3,3'-Dimethyl-4,4'-Bisphenylendiisocyanat, 3,3'-Dimethyl-Diisocyanatodiphenylmethan, die Isomerenmischungen 2,4'- und 4-4'-Diisocyanatodiphenylmethan und C₃₆-Dimerdiisocyanat.

Verbindungen der allgemeinen Formel (V) werden unter Bedingungen hergestellt, wie sie dem Fachmann bekannt sind. Die Reaktionstemperatur bei der Kondensationsreaktion der Dicarbonsäuren mit Diaminen oder Diolen liegt bevorzugt bei 100 bis 250 °C, besonders bevorzugt bei 140 bis 200 °C. Das Verhältnis von Diamin und Polycarbonsäure wird so gewählt, dass auf n Äquivalente Polycarbonsäure (n + 1) Äquivalente Diamin verwendet werden, so dass das Kondensationsprodukt eine Aminzahl von bevorzugt 5 bis 180 und besonders bevorzugt von 15 bis 100, bezogen auf 100 % aktive Substanz, aufweist.

Als Verbindungen der allgemeinen Formel (VI) finden vorzugsweise, wenn X für O steht, Acrylsäure sowie deren Salze, wie beispielsweise Natriumacrylat, Kaliumacrylat oder Ammoniumacrylat, und Acrylsäurealkylester von geradkettigen, verzweigten oder cycloaliphatischen Alkoholen mit 1 bis 22 C-Atomen wie beispielsweise Methylacrylat, Ethylacrylat, n-Propylacrylat, iso-Propylacrylat, n-Butylacrylat, iso-Butylacrylat, *tert.*-Butylacrylat, Pentylacrylat, Hexylacrylat, 2-Ethylhexylacrylat, n-Octylacrylat, iso-Octylacrylat, 3,5,5-Trimethylhexylacrylat, Octyldecylacrylat, Isodecylacrylat, Laurylacrylat, Tridecylacrylat, C16/C18 Alkylacrylat, Stearylacrylat, Behenylacrylat, Cyclohexylacrylat, Tetrahydrofurfurylacrylat, 2-Norbornylacrylat, Isobornylacrylat, Dicyclopentadienylacrylat, Dihydrodicyclopentadienylacrylat und Benzylacrylat Verwendung. Ebenfalls umfasst sind Acrylsäurearylester wie beispielsweise Phenylacrylat oder 2-Phenoxyethylacrylat. Der Einsatz von Acrylsäurehydroxyalkylester geradkettiger, verzweigter oder cycloaliphatischer Diole mit 2 bis 36 C-Atomen, wie beispielsweise 2-Hydroxyethylacrylat, 3-Hydroxypropylacrylat, 4-Hydroxybutylacrylat ist ebenfalls möglich, wie auch die Verwendung von Caprolacton- und/oder Valerolactonmodifizierten Hydroxyalkylacrylaten mit einem mittleren Molekulargewicht Mₙ von 220 bis 1200, wobei die Hydroxyacrylate bevorzugt von geradkettigen, verzweigten oder cycloaliphatischen Diolen mit 2 bis 8 C-Atomen abgeleitet sind. Polyoxyalkylenacrylate, wie Polyoxyethylenacrylate, Polyoxypropylenacrylate, oder Polyoxybutylenacrylate, gemischte Polyoxyethylen-Polyoxypropylenacrylate oder Mischungen von Polyoxyethylenacrylaten und Polyoxypropylenacrylaten mit höherhomologen Oxyalkylenacrylaten oder mit Styroloxid mit 5 bis 100 C-Atomen finden sowohl OH-funktionell als auch alkyl-, allyl oder aralkylverkappt mit 1 bis 22 C-Atomen wie z.B. Methoxy-, Ethoxy, Lauroxy- oder Stearoxypolyoxyethylenacrylat, Octoxy-, Stearoxy- oder Allyloxypolyoxyethylen-Polyoxypropylenacrylat oder Nonylphenoxypolyoxyalkylenacrylat Verwendung. Aminoalkylacrylate wie beispielsweise N,N-Dimethylaminoethylacrylat oder N,N'-Dimethylaminopropylacrylat können ebenfalls verwendet werden.

Für den Fall, dass in den Verbindungen der allgemeinen Formel (VI) X für NH steht, können außer Acrylamid auch substituierte Acrylamide wie z.B. Acrylsäure-tert.-butylamid, Acrylsäureisopropylamid, N-Methylolacrylsäureamid, N-Butoxymethylacrylsäureamid, N-Isobutoxymethylacrylsäureamid, N,N'-Dimethylaminopropylacrylsäureamid, Acrylsäurephenylamid und 2-Acrylamido-2-methyl-1-propansulfonsäure (AMPS) sowie dessen Salze Verwendung finden. Steht X für NR, so kommt beispielsweise N,N'-Acrylsäuredimethylamid zum Einsatz. Mischungen der einzelnen Acrylsäureester untereinander und zusammen mit den Acrylsäureamiden sind ebenfalls erfindungsgemäß.

Als Verbindungen der allgemeinen Formel (VII) kommen vorzugsweise folgende Monoisocyanate in Betracht: Methylisocyanat, Ethylisocyanat, Propylisocyanat, n-Butylisocyanat, tert.-Butylisocyanat, Isobutylisocyanat, Pentylisocyanat, Neopentylisocyanat, 2-Ethyl-hexylisocyanat, Octylisocyanat, Cyclohexylisocyanat, Phenylisocyanat, Toloylisocyanat, 1-Naphthylisocyanat, 2-Naphthylisocyanat, Stearylisocyanat sowie die aus der US 6,420,466 bekannten isocyanathaltigen Monoaddukte der allgemeinen Formel OCN-C₆H₃(CH₃)-NHCOO-R⁸, wobei R⁸ vorzugsweise für einen (C₁-C₂₂)Alkyl-, Aryl-, (C₇-C₁₂)Aralkyl-, oder (C₅-C₁₂)Cycloalkylrest, einen (C₁-C₂₂)Alkoxy-, (C₅-C₁₂)Cycloalkoxy- oder (C₇-C₁₂)Aralkoxypolyoxyalkylen-Rest, wobei es sich bei dem Oxyalkylen-Rest um Ethylen-, Propylen- oder Butylenoxid, einschließlich deren Mischungen, handelt und Teile des Oxyalkylen-Restes durch Styroloxid substituiert sein können oder einen (C₁-C₂₂)Alkanoi-, (C₅-C₁₂)Cycloalkanol-, (C₇-C₁₂)Aralkanolgestarteten oder einen (C₁-C₂₂)Alkoxy-, (C₅-C₁₂)Cycloalkoxy-, oder (C₇-C₁₂)Aralkoxypolyoxyalkylengestarteten Polyester auf Basis von z.B. (C₁-C₁₈)Hydroxycarbonsäuren oder Lactonen wie z.B. β-Propiolacton, δ-Valerolacton, ε-Caprolacton oder (C₁-C₆)Alkylsubstituiertem ε-Caprolacton, einschließlich deren Mischungen steht. Die einzelnen Monoisocyanate können auch in Mischungen eingesetzt werden.

Die Additionsreaktion zwischen den Verbindungen der allgemeinen Formel (V) und (VI) wird bevorzugt bei einer Reaktionstemperatur von 60 bis 100 °C, besonders bevorzugt 70 bis 90°C durchgeführt. Dabei wird das Verhältnis zwischen (V) und (VI) so gewählt, dass auf 1 mol Verbindung (V) wenn in Verbindung (II) R¹ für H steht, bevorzugt 1,8 mol bis 2,2 mol, besonders bevorzugt 1,9 mol bis 2,1 mol, und ganz besonders bevorzugt 2 mol der Verbindung der allgemeinen Formel (VI) und für den Fall, dass in Verbindung (II) R¹ für -(CH₂)₂-CO-X-R⁵ steht, bevorzugt 3,8 mol bis 4,2 mol, besonders bevorzugt 3,9 mol bis 4,1 mol, und ganz besonders bevorzugt 4 mol Verbindung (VI) eingesetzt wird. Für den Fall, dass in Verbindung (II) R¹ = -(CH₂)₂-CO-X-R⁵ ist, können die Acrylsäureester und -amide einzeln oder in Mischungen gleichzeitig oder sukzessive zu Verbindung (V) gegeben werden. Die Reaktion kann in Gegenwart oder Abwesenheit von Lösemitteln durchgeführt werden. Als Lösemittel geeignet sind alle aliphatischen, aromatischen, protischen und aprotischen Lösemittel wie beispielsweise Methoxypropylacetat, Cyclohexan, Toluol, Xylol, höhersiedende Alkylbenzole oder Isoparaffine, N-Methylpyrrolidon oder N-Ethylpyrrolidon, aber auch Alkohole wie Ethanol, Propanol, i-Butanol oder Glykole wie beispielsweise Butylglykol. Auch Mischungen von Lösemitteln lassen sich verwenden.

Für den Fall, dass in der Verbindung der allgemeinen Formel (II) R¹ für CONH-R' steht, wobei R' für R⁸ oder C₆H₃(CH₃)-NHCOO-R⁸ steht, wird bei einer Reaktionstemperatur von 50 bis 100 °C, besonders bevorzugt von 60 bis 80 °C zu 1 mol des Addukts aus Verbindung (V) und (VI) vorzugsweise 1,8 mol bis 2,2 mol, besonders bevorzugt 1,9 mol bis 2,1 mol, ganz besonders bevorzugt 2 mol der Verbindung der allgemeinen Formel (VII) gegeben. Die Monoisocyanate können gleich oder unterschiedlich sein. Diese Reaktion kann mit oder ohne Katalysator durchgeführt werden. Als Katalysatoren sind sowohl zinnorganische Verbindungen wie beispielsweise Dibutylzinndilaurat (DBTL) als auch tertiäre Amine wie beispielsweise Diazabicyclo[2.2.2]octan (DABCO) geeignet. Die Reaktion kann mit oder ohne Lösemittel durchgeführt werden. Als Lösemittel sind alle aliphatischen, aromatischen und aprotischen Lösemittel wie beispielsweise Methoxypropylacetat, Cyclohexan, Toluol, Xylol, höhersiedende Alkylbenzole oder Isoparaffine, N-Methylpyrrolidon oder N-Ethylpyrrolidon geeignet. Auch Mischungen von Lösemitteln lassen sich verwenden.

Verbindungen der allgemeinen Formel (III)

R⁵-X-CO-(CH₂)₂-NR¹-R²-[Y-R³-Y-R⁴]ₐ-Y-R⁶ (III)

worin R¹ für H, (CH₂)₂-CO-X-R⁵ oder CONH-R' steht und R' für R⁸ oder C₆H₃(CH₃)-NHCOO-R⁸ steht,
lassen sich beispielsweise herstellen, indem eine oder mehrere Verbindungen der allgemeinen Formel (VIII):

H₂N-R²-[Y-R³-Y-R⁴]ₐ-Y-R⁶ (VIII)

wobei R², R³ und R⁴ unabhängig voneinander für einen (C₂-C₄₀)Alkylen-, (C₃-C₄₀)Alkenylen-, (C₅-C₄₀)Cycloalkylen, Arylen-, (C₇-C₄₀)Aralkylen- oder Polyoxyalkylen-Rest stehen, wobei es sich bei dem Oxyalkylen-Rest um Ethylen-, Propylen- oder Butylenoxid, einschließlich deren Mischungen, handelt und Teile des Oxyalkylen-Restes durch Styroloxid substituiert sein können, oder einen Polyester-Rest auf Basis von z.B. (C₁-C₁₈)Hydroxycarbonsäuren oder Lactonen wie z.B. β-Propiolacton, δ-Valerolacton, ε-Caprolacton oder (C₁-C₆)Alkylsubstituiertem ε-Caprolacton, einschließlich Mischungen davon, steht,
Y für eine oder mehrere der folgenden Gruppen COO, OCO, NHCO, CONH, NHCOO, OOCNH, NHCONH steht, und
a für eine Zahl von 1 bis 19 steht, und
R⁶ für einen (C₁-C₃₀)Alkyl-, (C₃-C₂₂)Alkenyl-, Hydroxyalkyl-, (C₄-C₁₃)Cycloalkyl-, Aryl- oder (C₇-C₁₂)Aralkyl-Rest steht,
mit einer oder mehreren Verbindungen der allgemeinen Formel (VI):

R⁵-X-CO-CH=CH₂ (VI)

wobei R⁵ für H, einen (C₁-C₂₂)Alkyl-, Aryl-, (C₇-C₁₂)Aralkyl-, (C₅-C₁₂)Cycloalkyl-, Hydroxyalkyl-, oder N,N'-Dialkylamino-Rest, einen Hydroxy-, (C₁-C₂₂)Alkoxy-, (C₅-C₁₂)Cycloalkoxy-, oder (C₇-C₁₂)Aralkoxypolyoxyalkylen-Rest, wobei es sich bei dem Oxyalkylenrest um Ethylen-, Propylen- oder Butylenoxid, einschließlich deren Mischungen, handelt und Teile des Oxyalkylen-Restes durch Styroloxid substituiert sein können oder einen (C₁-C₂₂)Alkanol-, (C₅-C₁₂)Cycloalkanol-, (C₇-C₁₂)Aralkanolgestarteten oder einen (C₁-C₂₂)Alkoxy-, (C₅-C₁₂)Cycloalkoxy-, oder (C₇-C₁₂)Aralkoxypolyoxyalkylengestarteten Polyester auf Basis von z.B. (C₁-C₁₈)Hydroxycarbonsäuren oder Lactonen wie z.B. β-Propiolacton, δ-Valerolacton, ε-Caprolacton oder (C₁-C₆)Alkylsubstituiertem ε-Caprolacton, einschließlich Mischungen davon, steht,
X für O, NH oder NR⁹ steht, und
R⁹ für (C₁-C₂₂)Alkyl-, Aryl-, (C₇-C₁₂)Aralkyl-, Hydroxyalkyl-, (C₅-C₁₂)Cycloalkyl-Rest steht, umgesetzt werden
und anschließend das Produkt gegebenenfalls mit Verbindungen der allgemeinen Formel (VII):

R'-NCO (VII)

wobei R' für R⁸ oder -C₆H₃(CH₃)-NHCOO-R⁸ steht und
R⁸ für einen (C₁-C₂₂)Alkyl-, Aryl-, (C₇-C₁₂)Aralkyl-, oder (C₅-C₁₂)Cycloalkyl-Rest, einen (C₁-C₂₂)Alkoxy-, (C₅-C₁₂)Cycloalkoxy-, oder (C₇-C₁₂)Aralkoxypolyoxyalkylen-Rest, wobei es sich bei dem Oxyalkylen-Rest um Ethylen-, Propylen- oder Butylenoxid, einschließlich deren Mischungen, handelt und Teile des Oxyalkylen-Restes gegebenenfalls durch Styroloxid substituiert sind, einen (C₁-C₂₂)Alkanol-, (C₅-C₁₂)Cycloalkanol-, oder (C₇-C₁₂)Aralkanolgestarteten oder einen (C₁-C₂₂)Alkoxy-, (C₆-C₁₂)Cycloalkoxy-, oder (C₇-C₁₂)Aralkoxypolyoxyalkylengestarteten Polyester auf Basis von z.B. (C₁-C₁₈)Hydroxycarbonsäuren oder Lactonen wie z.B. β-Propiolacton, δ-Valerolacton, ε-Caprolacton oder (C₁-C₆)Alkylsubstituiertem ε-Caprolacton, einschließlich Mischungen davon, steht,
umgesetzt wird.

Die Zwischenverbindungen der allgemeinen Formel (VIII) werden unter Bedingungen hergestellt, wie sie dem Fachmann bekannt sind und sind beispielsweise dadurch erhältlich, dass man eine Mischung aus Mono- und Polycarbonsäuren, bevorzugt Dicarbonsäuren, und/oder Dicarbonsäureanhydriden, mit Diaminen, vorzugsweise bei Temperaturen von 100 bis 250 °C, besonders bevorzugt 140 bis 200 °C unter Wasserabscheidung umsetzt. Bevorzugt lassen sich Verbindungen der allgemeinen Formel (VIII) dadurch herstellen, dass zuerst die Dicarbonsäure und/oder das Dicarbonsäureanhydrid mit dem Diamin bei Temperaturen von 100 bis 250 °C, besonders bevorzugt 140 bis 200 °C unter Wasserabscheidung zum Kondensationsprodukt der allgemeinen Formel (V) mit einer Aminzahl von bevorzugt 5 bis 180 und besonders bevorzugt von 15 bis 100, bezogen auf 100 % aktive Substanz, umgesetzt wird und anschließend bei Temperaturen von 100 bis 250 °C, besonders bevorzugt 140 bis 200 °C, Verbindung (V) mit der Monocarbonsäure unter Wasserabscheidung zu Verbindung (VIII) umgesetzt wird, wobei dieses Kondensationsprodukt eine Aminzahl von bevorzugt 3 bis 80 und besonders bevorzugt von 8 bis 50, bezogen auf 100 % aktive Substanz, aufweist. Das Verhältnis Diamin zu Polycarbonsäure zu Monocarbonsäure beträgt 3 zu 2 zu 1 bis 20 zu 19 zu 1, besonders bevorzugt 3 zu 2 zu 1 bis 8 zu 7 zu 1.

Die Mono-, bzw. Polycarbonsäuren können teilweise durch Mono-, bzw. Diisocyanate und die Diamine teilweise durch Diole ersetzt werden, wobei dann Ester-, Urethan- und/oder Harnstoffgruppen neben den bevorzugten Amidgruppierungen in den Verbindungen der allgemeinen Formel (VIII) vorliegen können.

Bei den Monocarbonsäuren handelt es sich um gesättigte, einfach bis mehrfach ungesättigte, lineare und verzweigte aliphatische Carbonsäuren wie z.B. Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Behensäure, Lignocerinsäure, Cerotinsäure, Melissinsäure, Lauroleinsäure, Myristoleinsäure, Palmitoleinsäure, Petroselinsäure, Ölsäure, Elaidinsäure, Vaccensäure, Erucasäure, Linolsäure, Linolensäure, Arachidonsäure, Clupanodonsäure, Ricinensäure, α-Elaeostearinsäure, α-Parinarsäure, Kokosölfettsäure, Palmkernölfettsäure, Kokos/Palmkernölfettsäure, Palmölfettsäure, Cottonölfettsäure, Erdnussölfettsäure, Sojaölfettsäure, Sonnenblumenölfettsäure, Rapsölfettsäure und Talgfettsäure. Ebenso finden Ketocarbonsäuren wie beispielsweise Licansäure, als auch aromatische Monocarbonsäuren wie beispielsweise Benzoesäure Verwendung. Als Vertreter der Hydroxycarbonsäuren seien zum Beispiel Glykolsäure, 5-Hydroxyvaleriansäure, 6-Hydroxycapronsäure, Ricinolfettsäure, 12-Hydroxystearinsäure, 12-Hydroxydodecansäure, 5-Hydroxydodecansäure, 5-Hydroxydecansäure oder 4-Hydroxydecansäure genannt. Auch Mischungen der Monocarbonsäuren können eingesetzt werden.

Die Additionsreaktion zwischen den Verbindungen der allgemeinen Formeln (VIII) und (VI) wird vorzugsweise bei einer Reaktionstemperatur von 60 bis 100 °C, besonders bevorzugt von 70 bis 90°C durchgeführt. Dabei wird das Verhältnis zwischen den Verbindungen der allgemeinen Formeln (VIII) und (VI) vorzugsweise so gewählt, dass auf 1 mol Verbindung der allgemeinen Formel (VIII), wenn in dieser R¹ für H steht, 0,8 mol bis 1,2 mol, besonders bevorzugt 0,9 mol bis 1,1 mol, und ganz besonders bevorzugt 1 mol Verbindung der allgemeinen Formel (VI) und für den Fall, dass in der Verbindung der allgemeinen Formel (III) R¹ für (CH₂)₂-CO-X-R⁵ steht, vorzugsweise 1,8 mol bis 2,2 mol, besonders bevorzugt 1,9 mol bis 2,1 mol, und ganz besonders bevorzugt 2 mol Verbindung (VI) eingesetzt wird. Für den Fall, dass in Verbindung (III) R¹ für (CH₂)₂-CO-X-R⁵ steht, können die Acrysäureester undamide einzeln oder in Mischungen gleichzeitig oder sukzessive zur Verbindung der allgemeinen Formel (VIII) gegeben werden. Die Reaktion kann mit oder ohne Lösemittel durchgeführt werden. Als Lösemittel geeignet sind alle aliphatischen, aromatischen, protischen und aprotischen Lösemittel wie beispielsweise Methyoxypropylacetat, Cyclohexan, Toluol, Xylol, höhersiedende Alkylbenzole oder lsoparaffine. N-Methylpyrrolidon oder N-Ethylpyrrolidon, aber auch Alkohole wie Ethanol, Propanol, i-Butanol oder Glykole wie zum Beispiel Butylglykol sind ebenfalls geeignet. Auch Mischungen von Lösemitteln lassen sich verwenden.

Für den Fall, dass in den Verbindungen der Formel (III) R¹ für CONH-R' mit R'= R⁸ oder C₆H₃(CH₃)-NHCOO-R⁸ steht, wird vorzugsweise bei einer Reaktionstemperatur von vorzugsweise 50 bis 100 °C, besonders bevorzugt 60 bis 80 °C auf 1 mol des Adduktes aus den Verbindungen der allgemeinen Formeln (VIII) und (VI) vorzugsweise 0,8 mol bis 1,2 mol, besonders bevorzugt 0,9 mol bis 1,1 mol, und ganz besonders bevorzugt 1 mol der Verbindung der allgemeinen Formel (VII) gegeben. Die Monoisocyanate können gleich oder unterschiedlich sein. Die Reaktion kann mit oder ohne Katalysator durchgeführt werden. Als Katalysatoren sind sowohl zinnorganische Verbindungen wie beispielsweise DBTL als auch tertiäre Amine wie zum Beispiel DABCO geeignet. Diese Reaktion kann mit oder ohne Lösemittel durchgeführt werden. Als Lösemittel geeignet sind alle aliphatischen, aromatischen und aprotischen Lösemittel wie beispielsweise Methoxypropylacetat, Cyclohexan, Toluol, Xylol, höhersiedende Alkylbenzole oder Isoparaffine, N-Methylpyrrolidon oder N-Ethylpyrrolidon. Auch Mischungen von Lösemitteln lassen sich verwenden.

Verbindungen der allgemeinen Formel (IV)

R⁶-Y-[R⁴-Y-R³-Y]ₐ-R²-NR¹-(CH₂)₂-CO-X-R⁷-X-CO-(CH₂)₂-NR¹-R²-[Y-R³-Y-R⁴]ₐ-Y-R⁶ (IV)

mit R¹ = -H, -(CH₂)₂-CO-X-R⁵, -CONH-R' mit R'= R⁸ oder-C₆H₃(CH₃)-NHCOO-R⁸
lassen sich beispielsweise herstellen, indem eine oder mehrere Verbindungen der allgemeinen Formel (VIII):

H₂N-R²-[Y-R³-Y-R⁴]ₐ-Y-R⁶ (VIII)

wobei R², R³ und R⁴ unabhängig voneinander für einen (C₂-C₄₀)Alkylen-, (C₃-C₄₀)Alkenylen-, (C₅-C₄₀)Cycloalkylen, Arylen-, (C₇-C₄₀)Aralkylen- oder Polyoxyalkylen-Rest stehen, wobei es sich bei dem Oxyalkylen-Rest um Ethylen-, Propylen- oder Butylenoxid, einschließlich deren Mischungen, handelt und Teile des Oxyalkylen-Restes durch Styroloxid substituiert sein können, oder einen Polyester-Rest auf Basis von z.B. (C₁-C₁₈)Hydroxycarbonsäuren oder Lactonen wie z.B. β-Propiolacton, δ-Valerolacton, ε-Caprolacton oder (C₁-C₆)Alkylsubstituiertem ε-Caprolacton, einschließlich Mischungen davon, steht,
Y für eine oder mehrere der folgenden Gruppen COO, OCO, NHCO, CONH, NHCOO, OOCNH, NHCONH steht, und
a für eine Zahl von 1 bis 19 steht, und
R⁶ für einen (C₁-C₃₀)Alkyl-, (C₃-C₂₂)Alkenyl-, Hydroxyalkyl-, (C₄-C₁₃)Cycloalkyl-, Aryl- oder (C₇-C₁₂)Aralkylrest steht,
mit einer oder mehreren Verbindungen der allgemeinen Formel (IX):

H₂C=HC-CO-X-R⁷-X-CO-CH=CH₂ (IX)

wobei R⁷ für einen (C₁-C₁₈)Alkylen-, (C₃-C₁₈)Alkenylen-, (C₅-C₁₂)Cycloalkylen-, Arylen-, (C₇-C₁₅)Aralkylen- oder Polyoxyalkylen-Rest, wobei es sich bei dem Oxyalkylen-Rest um Ethylen-, Propylen- oder Butylenoxid, einschließlich deren Mischungen, handelt und Teile des Oxyalkylen-Restes durch Styroloxid substituiert sein können, oder einen Polyester-Rest auf Basis von z.B. (C₁-C₁₈)Hydroxycarbonsäuren oder Lactonen wie z.B. β-Propiolacton, δ-Valerolacton, ε-Caprolacton oder (C₁-C₆)Alkylsubstituiertem ε-Caprolacton, einschließlich Mischungen davon, steht
X für O, NH oder NR⁹ steht, und
R⁹ für (C₁-C₂₂)Alkyl-, Aryl-, (C₇-C₁₂)Aralkyl-, Hydroxyalkyl-, (C₅-C₁₂)Cycloalkylrest steht, umgesetzt werden
und anschließend das Produkt gegebenenfalls
(a) mit einer oder mehreren Verbindungen der allgemeinen Formel (VI):

   R⁵-X-CO-CH=CH₂ (VI)

   wobei R⁵ für H, einen (C₁-C₂₂)Alkyl-, Aryl-, (C₇-C₁₂)Aralkyl-, (C₅-C₁₂)Cycloalkyl-, Hydroxyalkyl-, oder N,N'-Dialkylamino-Rest, einen Hydroxy-, (C₁-C₂₂)Alkoxy-, (C₅-C₁₂)Cycloalkoxy-, oder (C₇-C₁₂)Aralkoxypolyoxyalkylen-Rest, wobei es sich bei dem Oxyalkylen-Rest um Ethylen-, Propylen- oder Butylenoxid, einschließlich deren Mischungen, handelt und Teile des Oxyalkylenrestes durch Styroloxid substituiert sein können oder einen (C₁-C₂₂)Alkanol-, (C₅-C₁₂)Cycloalkanol-, (C₇-C₁₂)Aralkanolgestarteten oder einen (C₁-C₂₂)Alkoxy-, (C₅-C₁₂)Cycloalkoxy-, oder (C₇-C₁₂)Aralkoxypolyoxyalkylengestarteten Polyester auf Basis von z.B. (C₁-C₁₈)Hydroxycarbonsäuren oder Lactonen wie z.B. β-Propiolacton, δ-Valerolacton, ε-Caprolacton oder (C₁-C₆)Alkylsubstituiertem ε-Caprolacton, einschließlich Mischungen davon, steht,
   X für O, NH oder NR⁹ steht, und
   R⁹ für (C₁-C₂₂)Alkyl-, Aryl-, (C₇-C₁₂)Aralkyl-, Hydroxyalkyl-, (C₅-C₁₂)Cycloalkyl-Rest steht,
   und/oder
(b) einer oder mehreren Verbindungen der allgemeinen Formel (VII):

   R'-NCO (VII)
wobei R' für R⁸ oder -C₆H₃(CH₃)-NHCOO-R⁸ steht und
R⁸ für einen (C₁-C₂₂)Alkyl-, Aryl-, (C₇-C₁₂)Aralkyl-, oder (C₅-C₁₂)Cycloalkyl-Rest, einen (C₁-C₂₂)Alkoxy-, (C₅-C₁₂)Cycloalkoxy-, oder (C₇-C₁₂)Aralkoxypolyoxyalkylen-Rest, wobei es sich bei dem Oxyalkylen-Rest um Ethylen-, Propylen- oder Butylenoxid, einschließlich deren Mischungen, handelt und Teile des Oxyalkylenrestes gegebenenfalls durch Styroloxid substituiert sind, einen (C₁-C₂₂)Alkanol-, (C₅-C₁₂)Cycloalkanol-, oder (C₇-C₁₂)Aralkanolgestarteten oder einen (C₁-C₂₂)Alkoxy-, (C₆-C₁₂)Cycloalkoxy-, oder (C₇-C₁₂)Aralkoxypolyoxyalkylengestarteten Polyester auf Basis von z.B. (C₁-C₁₈)Hydroxycarbonsäuren oder Lactonen wie z.B. β-Propiolacton, δ-Valerolacton, ε-Caprolacton oder (C₁-C₆)Alkylsubstituiertem ε-Caprolacton, einschließlich Mischungen davon, steht,
umgesetzt wird.

Bei den Verbindungen der allgemeinen Formel (IX) handelt es sich, wenn X für O steht, vorzugsweise um Diacrylate geradkettiger, verzweigter oder cycloaliphatischer Diole mit 2 bis 36 C-Atomen, wie beispielsweise Ethandioldiacrylat, 1,3-Butandioldiacrylat, 1,4- Butandioldiacrylat, 1,6-Hexandioldiacrylat, 1,10-Decandioldiacrylat oder 2,2-Dimethyl-1,3-propandioldiacrylat. Oligo- und Polyoxyalkylendiacrylate mit 10 bis 100 C-Atomen wie beispielsweise Diethylenglykoldiacrylat, Triethylenglykoldiacrylat, Tetraethylenglykoldiacrylat, Dipropylenglykoldiacrylat, Tripropylenglykoldiacrylat, Polyoxyethylendiacrylat, Polyoxypropylendiacrylat, Polyoxybutylendiacrylat, Polyoxyethylen-Polyoxybutylendiacrylat, Polyoxypropylen-Polyoxybutylendiacrylat, Polyoxyethylen-Polyoxypropylen-Polyoxyethylendiacrylat sind ebenso eingeschlossen wie Urethandiacrylat, Bisphenol A Epoxid Diacrylat, ethoxyliertes und/oder propoxyliertes Bisphenol A Diacrylat mit 1 bis 15 Alkoxygruppen, ethoxyliertes oder propoxyliertes 2,2-Dimethyl-1,3-propandioldiacrylat. Polyesterdiacrylate wie beispielsweise Polylactondiacrylate auf Basis Caprolacton und/oder Valerolacton und gemischte Polyether-Polyesterdiacrylate sind ebenfalls als Verbindungen der allgemeinen Formel (IX) einsetzbar.

Für den Fall, dass X für NH steht, finden vorzugsweise Bisacrylsäureamide wie beispielsweise N, N'-Methylenbisacrylsäureamid oder N, N'-(1,2-Dihydroxyethylen)bisacrylsäureamid Verwendung. Die Diacrylate lassen sich auch in Mischungen untereinander oder zusammen mit den Bisacrylsäureamiden einsetzen, ebenso können geringe Mengen an Triacrylaten wie zum Beispiel Trimethylolpropantriacrylat oder alkoxyliertes Trimethylolpropantriacrylat verwendet werden.

Die Additionsreaktion zwischen den Verbindungen der allgemeinen Formel (VIII) und (IX) wird vorzugsweise bei einer Reaktionstemperatur von 60 bis 100 °C, besonders bevorzugt von 70 bis 90°C durchgeführt. Dabei wird das Verhältnis zwischen den Verbindungen der allgemeinen Formeln (VIII) und (IX) vorzugsweise so gewählt, dass auf 1 mol der Verbindung der allgemeinen Formel (IX), wenn in dieser R¹ für H steht, vorzugsweise 1,8 mol bis 2,2 mol, besonders bevorzugt 1,9 mol bis 2,1 mol, und ganz besonders bevorzugt 2 mol Verbindung (VIII) eingesetzt wird. Wenn in der Verbindung der allgemeinen Formel (IV) R¹ für (CH₂)₂-CO-X-R⁵ steht, wird vorzugsweise auf 1 mol des Adduktes aus den Verbindungen der allgemeinen Formeln (VIII) und (IX) bei einer Reaktionstemperatur von vorzugsweise 60 bis 100 °C, besonders bevorzugt 70 bis 90°C, vorzugsweise 1,8 mol bis 2,2 mol, besonders bevorzugt 1,9 mol bis 2,1 mol, und ganz besonders bevorzugt 2 mol Verbindung (VI) eingesetzt. Die Reaktion kann mit oder ohne Lösemittel durchgeführt werden. Als Lösemittel geeignet sind alle aliphatischen, aromatischen, protischen und aprotischen Lösemittel wie beispielsweise Methoxypropylacetat, Cyclohexan, Toluol, Xylol, höhersiedende Aromaten oder lsoparaffine. N-Methylpyrrolidon oder N-Ethylpyrrolidon, aber auch Alkohole wie Ethanol, Propanol, i-Butanol oder Glykole wie beispielsweise Butylglykol sind ebenfalls geeignet. Auch Mischungen von Lösemitteln lassen sich verwenden.

Für den Fall, dass in den Verbindung der allgemeinen Formel (IV) R¹ für CONH-R' mit R'= R⁸ oder -C₆H₃(CH₃)-NHCOO-R⁸ steht, wird bei einer Reaktionstemperatur von vorzugsweise 50 bis 100 °C, besonders bevorzugt von 60 bis 80°C auf vorzugsweise 1 mol des Adduktes aus den Verbindung der allgemeinen Formeln (VIII) und (IX) vorzugsweise 1,8 mol bis 2,2 mol, besonders bevorzugt 1,9 mol bis 2,1 mol, und ganz besonders bevorzugt 2 mol der Verbindung der allgemeinen Formel (VII) gegeben. Die Monoisocyanate können gleich oder unterschiedlich sein. Diese Reaktion kann mit oder ohne Katalysator durchgeführt werden. Als Katalysatoren sind sowohl zinnorganische Verbindungen wie beispielsweise DBTL als auch tertiäre Amine wie zum Beispiel DABCO geeignet. Diese Reaktion kann mit oder ohne Lösemittel durchgeführt werden. Als Lösemittel geeignet sind alle aliphatischen, aromatischen und aprotischen Lösemittel wie beispielsweise Methoxypropylacetat, Cyclohexan, Toluol, Xylol, höhersiedende Alkylbenzole oder Isoparaffine, N-Methylpyrrolidon oder N-Ethylpyrrolidon. Auch Mischungen von Lösemitteln lassen sich verwenden.

Des Weiteren betrifft die vorliegende Erfindung die Verwendung der erfindungsgemäßen amidhaltigen Polymere beziehungsweise der durch das erfindungsgemäße Verfahren erhaltenen amidhaltigen Polymere als Rheologiesteuerungsadditive, insbesondere in lösungsmittelhaltigen und lösungsmittelfreien Lacken auf der Basis von Bindemitteln wie z.B. Polyurethanen (1K und 2K), Polyacrylaten, Polyester-, Alkyd- und Epoxidharzen, PVC-Plastisolen und -Organosolen, Beschichtungen auf Epoxidbasis und ungesättigten Polyesterharzen. Unter Lacken können auch Nagellacke verstanden werden, wie sie beispielsweise in der Patentschrift US 6,156,325 beschrieben sind.

Die Einsatzmenge der erfindungsgemäßen amidhaltigen Polymere beträgt üblicherweise 0,05 bis 5,0 Gew.-% Wirksubstanz, bevorzugt 0,1 bis 3,0 Gew.-% Wirksubstanz und besonders bevorzugt 0,2 bis 2,0 Gew.-% Wirksubstanz, bezogen auf das Gewicht der Gesamtformulierung.

Weiterer Gegenstand der Erfindung sind gehärtete und ungehärtete Polymer-Zusammensetzungen enthaltend ein oder mehrere der erfindungsgemäßen amidhaltigen Polymere beziehungsweise der durch das erfindungsgemäße Verfahren erhaltenen amidhaltigen Polymere.

### BEISPIELE

### Beispiel 1:

In einem 1-Liter 3-Halskolben mit Rührer, Wasserabscheider und Thermometer werden nacheinander 226,4 g (0,4 mol) Dimersäure Pripol^{™} 1006 (Dimerisierte Fettsäure, hydriert, Uniqema), 69,6 g (0,6 mol) Hexamethylendiamin und 127g Shellsol A (hochsiedender aromatischer Kohlenwasserstoff, Shell Chemicals) eingewogen und langsam auf 170°C erhitzt. Das bei der Reaktion langsam freiwerdende Wasser wird azeotrop über den Wasserabscheider abgetrennt. Das Kondensationsprodukt besitzt eine Aminzahl von ca. 54. Danach wird die Reaktionsmischung auf 50°C abgekühlt.

### Beispiel 2:

In einem 1-Liter 3-Halskolben mit Rührer, Wasserabscheider und Thermometer werden nacheinander 226,4g (0,4 mol) Dimersäure Pripol^{™} 1006 (Uniqema), 55,7g (0,48 mol) Hexamethylendiamin und 121g Shellsol D40 (dearomatisierter Kohlenwasserstoff, Shell Chemicals) eingewogen und langsam auf 170°C erhitzt. Das bei der Reaktion langsam freiwerdende Wasser wird azeotrop über den Wasserabscheider abgetrennt. Das Kondensationsprodukt besitzt eine Aminzahl von ca. 23. Danach wird die Reaktionsmischung auf 50°C abgekühlt.

### Beispiel 3:

In einem 1-Liter 3-Halskolben mit Rührer, Wasserabscheider und Thermometer werden nacheinander 226,4 g (0,4 mol) Dimersäure Pripol^{™} 1006 (Uniqema), 69,6 g (0,6 mol) Hexamethylendiamin und 152 g Shellsol D40 eingewogen und langsam auf 170°C erhitzt. Das bei der Reaktion langsam freiwerdende Wasser wird azeotrop über den Wasserabscheider abgetrennt. Das Kondensationsprodukt besitzt eine Aminzahl von ca. 51. Anschließend werden 57,6 g (0,2 mol) Tallölfettsäure (Mischung von Monocarbonsäuren mit einem hohen Gehalt an Öl- und Linolsäure und ca. 2% Harzsäuren (Abietinsäure), Arizona Chemical GmbH) zugegeben und das bei dieser Reaktion freiwerdende Wasser azeotrop über den Wasserabscheider abgetrennt. Dieses Kondensationsprodukt besitzt eine Aminzahl von ca. 23. Danach wird die Reaktionsmischung auf 50°C abgekühlt.

### Beispiel 4:

In einem 1-Liter 3-Halskolben mit Rührer, Wasserabscheider und Thermometer werden nacheinander 232 g (0,4 mol) Dimersäure Pripol^{™} 1017 (Dimerisierte Fettsäure, ungesättigt, Uniqema), 55,7 g (0,48 mol) Hexamethylendiamin und 133 g Cobersol B 60 (Isoparaffinisches Kohlenwasserstoffgemisch, C9 - C 12 lsoalkane, Cölner Benzin-Raffinerie) eingewogen und langsam auf 170°C erhitzt. Das bei der Reaktion langsam freiwerdende Wasser wird azeotrop über den Wasserabscheider abgetrennt. Das Kondensationsprodukt besitzt eine Aminzahl von ca. 22. Anschließend werden 22,8 g (0,08 mol) Stearinsäure zugegeben und das bei dieser Reaktion freiwerdende Wasser azeotrop über den Wasserabscheider abgetrennt. Dieses Kondensationsprodukt besitzt eine Aminzahl von ca. 10,5. Danach wird die Reaktionsmischung auf 50°C abgekühlt.

### Beispiel 5:

In einem 1-Liter 3-Halskolben mit Rührer, Wasserabscheider und Thermometer werden nacheinander 214,5 g (0,375 mol) Dimersäure Empol^{®} 1062 (Destillierte Dimerisierte Fettsäure, partiell hydriert, Cognis), 68,1 g (0,5 mol) m-Xylylendiamin und 121g Cobersol B 60 eingewogen und langsam auf 170°C erhitzt. Das bei der Reaktion langsam freiwerdende Wasser wird azeotrop über den Wasserabscheider abgetrennt. Das Kondensationsprodukt besitzt eine Aminzahl von ca. 35. Danach wird die Reaktionsmischung auf 50°C abgekühlt.

### Beispiel 6:

In einem 1-Liter 3-Halskolben mit Rührer, Wasserabscheider und Thermometer werden nacheinander 146 g (1 mol) Adipinsäure, 180 g (1,25 mol) Octamethylendiamin und 140 g Shellsol A eingewogen und langsam auf 170°C erhitzt. Das bei der Reaktion langsam freiwerdende Wasser wird azeotrop über den Wasserabscheider abgetrennt. Das Kondensationsprodukt besitzt eine Aminzahl von ca. 63. Danach wird die Reaktionsmischung auf 50°C abgekühlt.

### Beispiel 7:

In einem 500-mL 3-Halskolben mit Rührer, Rückflusskühler und Thermometer werden nacheinander 150,9 g (0,075 mol) des Kondensationsproduktes aus Beispiel 1 und 17 g (0,15 mol) N-Isopropylacrylamid eingewogen und auf 80°C erwärmt. Die Reaktionsmischung wird 3 Std. gerührt. Dann wird das Produkt mit i-Butanol auf 40% Festkörper verdünnt.

### Beispiel 8:

In einem 500-mL 3-Halskolben mit Rührer, Rückflusskühler und Thermometer werden nacheinander 143,4 g (0,03 mol) des Kondensationsproduktes aus Beispiel 2 und 13 g (0,06 mol) Isodecylacrylat eingewogen und auf 80°C erwärmt. Die Reaktionsmischung wird 3 Std. gerührt. Dann wird das Produkt mit i-Butanol auf 40% Festkörper verdünnt.

### Beispiel 9:

In einem 500-mL 3-Halskolben mit Rührer, Rückflusskühler und Thermometer werden nacheinander 143,4 g (0,03 mol) des Kondensationsproduktes aus Beispiel 2 und 16 g (0,06 mol) Polyoxyethylen 200 Acrylat (Blemmer^{®} AE 200, NOF) eingewogen und auf 80°C erwärmt. Die Reaktionsmischung wird 3 Std. gerührt. Dann wird das Produkt mit i-Butanol auf 40% Festkörper verdünnt.

### Beispiel 10:

In einem 500-mL 3-Halskolben mit Rührer, Rückflusskühler und Thermometer werden nacheinander 143,8 g (0,06 mol) des Kondensationsproduktes aus Beispiel 3 und 15 g (0,03 mol) Polyoxyethylen 400 Diacrylat (Sartomer^{™} 344, Cray Valley) eingewogen und auf 80°C erwärmt. Die Reaktionsmischung wird 3 Std. gerührt. Dann wird das Produkt mit i-Butanol auf 40% Festkörper verdünnt.

### Beispiel 11:

In einem 500-mL 3-Halskolben mit Rührer, Rückflusskühler und Thermometer werden nacheinander 143,8 g (0,06 mol) des Kondensationsproduktes aus Beispiel 3 und 4,6 g (0,03 mol) N,N'-Methylenbisacrylamid eingewogen und auf 80°C erwärmt. Die Reaktionsmischung wird 3 Std. gerührt. Dann wird das Produkt mit i-Butanol auf 40% Festkörper verdünnt.

### Beispiel 12:

In einem 500-mL 3-Halskolben mit Rührer, Rückflusskühler und Thermometer werden nacheinander 154,97 g (0,03 mol) des Kondensationsproduktes aus Beispiel 4 und 18 g (0,03 mol) Polyoxyethylen 400 Acrylat (Blemmer^{®} AE 400, NOF) eingewogen und auf 80°C erwärmt. Die Reaktionsmischung wird 3 Std. gerührt. Dann wird das Produkt mit i-Butanol auf 40% Festkörper verdünnt.

### Beispiel 13:

In einem 500-mL 3-Halskolben mit Rührer, Rückflusskühler und Thermometer werden nacheinander 154,97 g (0,03 mol) des Kondensationsproduktes aus Beispiel 4 und 9,7 g (0,03 mol) Stearylacrylat eingewogen und auf 80°C erwärmt. Die Reaktionsmischung wird 3 Std. gerührt. Dann wird das Produkt mit i-Butanol auf 40% Festkörper verdünnt.

### Beispiel 14:

In einem 500-mL 3-Halskolben mit Rührer, Rückflusskühler und Thermometer werden nacheinander 154,97 g (0,03 mol) des Kondensationsproduktes aus Beispiel 4 und 12,5 g (0,03 mol) Lauroxypolyoxyethylen 200 Acrylat (Blemmer^{®} ALE 200, NOF) eingewogen und auf 80°C erwärmt. Die Reaktionsmischung wird 3 Std. gerührt. Anschließend werden 9 g (0,03 mol) Stearylisocyanat zugegeben und weitere 2 h bei 80°C gerührt. Dann wird das Produkt mit i-Butanol auf 30% Festkörper verdünnt.

### Beispiel 15:

In einem 500-mL 3-Halskolben mit Rührer, Rückflusskühler und Thermometer werden nacheinander 153,77 g (0,05 mol) des Kondensationsproduktes aus Beispiel 5 und 34,4 g (0,1 mol) Tone^{™} M 100 (Additionsprodukt aus Hydroxyethylacrylat mit 2 mol ε-Caprolacton, DOW) eingewogen und auf 80°C erwärmt. Die Reaktionsmischung wird 3 Std. gerührt. Dann wird das Produkt mit i-Butanol auf 30% Festkörper verdünnt.

### Beispiel 16:

In einem 500-mL 3-Halskolben mit Rührer, Rückflusskühler und Thermometer werden nacheinander 82,86 g (0,05 mol) des Kondensationsproduktes aus Beispiel 6 und 24 g (0,1 mol) Laurylacrylat eingewogen und auf 80°C erwärmt. Die Reaktionsmischung wird 3 Std. gerührt. Anschließend werden 24,8 g (0,1 mol) des lsocyanatpräpolymeren der Formel OCN-C₆H₄(CH₃)-NHCOOC₄H₉ aus US 6,420,466 zugegeben und weitere 2 h bei 80°C gerührt. Dann wird das Produkt mit i-Butanol auf 30% Festkörper verdünnt.

### Anwendungsbeispiele

Weißlackrezeptur: Degalan Weißlack, 28% Bindemittel, 20% TiO₂

| | |
|---|---|
| Degalan^{®} LP 64/12, 35% in Shellsol A (Thermoplastisches Acrylat, 35 % angelöst in Shellsol A, Röhm GmbH) | 34,4 |
| Disperbyk^{®} 110 | 0,6 |
| Kronos 2160 (TiO₂) | 20 |

Dispergierung: 30 min. Dispermat, 40°C, 18 m/s Umfangsgeschwindigkeit (8000 U/min 4,5 cm Teflonscheibe) Glasperlen 1 mm 1:1 mit Mahlgut

| | |
|---|---|
| Degalan^{®} LP 64/12, 35% in Shellsol A | 45 |
| | 100 |

Additivdosierung: 1 Gew.-% Wirksubstanz, bezogen auf das Gewicht der Gesamtzusammensetzung

Einarbeitung: Additiv unter Rühren mit dem Dispermat zugeben Zahnscheibe 2,5 cm, 1000 U/min, 2 min

Abprüfung: Prüfung der rheologischen Wirksamkeit in Form der Ablaufgrenze. Dazu werden die additivierten Lacksysteme mit dem Stufen rakel 50 - 500 µm und 550 - 1000 µm auf Kontrastkarten 2801 von BYK Gardner mit einer automatischer Aufziehbank von BYK Gardner (Geschwindigkeit 3 cm/sec) appliziert. Die Kontrastkarten werden vertikal hängend getrocknet. Das Standvermögen wird in µm nass abgelesen. Dieses ist ein Maß für die rheologische Wirksamkeit. Die Ergebnisse sind in Tabelle 1 dargestellt.

**Tabelle 1**

| Additiv | Ablaufgrenze [µm] |
|---|---|
| Kontrolle (ohne Additiv) | 90 |
| Beispiel 7 | 700 |
| Beispiel 8 | 900 |
| Beispiel 9 | 850 |
| Beispiel 10 | 850 |
| Beispiel 11 | 1000 |
| Beispiel 12 | 600 |
| Beispiel 13 | 900 |
| Beispiel 14 | 650 |
| Beispiel 15 | 650 |
| Beispiel 16 | 550 |

## Patentansprüche

1. Amidhaltige Polymere der allgemeinen Formel (I)
A-X-CO-(CH₂)₂-NR¹-R²-[Y-R³-Y-R⁴]ₐ-B (I)
sowie deren Salze mit Carbonsäuren, Phosphorsäureestern und Sulfonsäuren, wobei
A für R⁵ oder R⁶-Y-[R⁴-Y-R³-Y]_{b}-R²-NR¹-(CH₂)₂-CO-X-R⁷ und
B für Y-R⁶ oder NR¹-(CH₂)₂-CO-X-R⁵ steht, und wobei
R¹ für H, (CH₂)₂-CO-X-R⁵ und/oder CONH-R' mit R' = R⁸ oder -C₆H₃(CH₃)-NHCOO-R⁸ steht,
R², R³, R⁴ und R⁷ unabhängig voneinander für einen (C₁-C₄₀)Alkylen-, (C₃-C₄₀)Alkenylen-, (C₅-C₄₀)Cycloalkylen-, Arylen-, (C₇-C₄₀)Aralkylen- oder Polyoxyalkylen-Rest oder für einen Polyesterrest stehen,
R⁵ für H, einen (C₁-C₂₂)Alkyl-, Aryl-, (C₇-C₁₂)Aralkyl-, (C₅-C₁₂)Cycloalkyl-, Hydroxyalkyl-, oder N,N'-Dialkylamino-Rest, einen Hydroxy-, (C₁-C₂₂)Alkoxy-, (C₅-C₁₂)Cycloalkoxy-, oder (C₇-C₁₂)Aralkoxy-Polyoxyalkylen-Rest, oder einen (C₁-C₂₂)Alkanol-, (C₅-C₁₂)Cycloalkanol-, (C₇-C₁₂)Aralkanol-gestarteten oder einen (C₁-C₂₂)Alkoxy-, (C₅-C₁₂)Cycloalkoxy-, oder (C₇-C₁₂)Aralkoxy-Polyoxyalkylen-gestarteten Polyester steht,
R⁶ für einen (C₁-C₃₀)Alkyl-, (C₃-C₂₂)Alkenyl-, Hydroxyalkyl-, (C₄-C₁₃)Cycloalkyl-, Aryl-oder (C₇-C₁₂)Aralkyl-Rest steht,
R⁸ für einen (C₁-C₂₂)Alkyl-, Aryl-, (C₇-C₁₂)Aralkyl-, oder (C₅-C₁₂)Cycloalkyl-Rest, einen (C₁-C₂₂)Alkoxy-, (C₅-C₁₂)Cycloalkoxy-, oder (C₇-C₁₂)Aralkoxy-Polyoxyalkylen-Rest, einen (C₁₋C₂₂)Alkanol-, (C₅-C₁₂)Cycloalkanol-, oder (C₇-C₁₂)Aralkanol-gestarteten oder einen (C₁-C₂₂)Alkoxy-, (C₆-C₁₂)Cycloalkoxy-, oder (C₇-C₁₂)Aralkoxy-Polyoxyalkylen-gestarteten Polyester steht,
X für gleiche oder verschiedene Reste O, NH oder NR⁹ steht,
R⁹ für einen (C₁-C₂₂)Alkyl-, Aryl-, (C₇-C₁₂)Aralkyl-, Hydroxyalkyl-, (C₅-C₁₂)CycloalkylRest steht,
Y für eine oder mehrere der folgenden Gruppen COO, OCO, NHCO, CONH, NHCOO, OOCNH, NHCONH steht und
a und b unabhängig voneinander für eine Zahl von 1 bis 19 stehen.

2. Amidhaltige Polymere nach Anspruch 1, wobei im Falle, dass einer oder mehrere der Reste R², R³, R⁴, R⁵, R⁷ und/oder R⁸ einen Polyoxyalkylen-Anteil enthalten, dieser Rest oder diese Reste aus Ethylenoxid, Propylenoxid und/oder Butylenoxid-Einheiten, statistisch oder blockweise angeordnet, aufgebaut sind, und gegebenenfalls einer oder mehrere der Ethylenoxid, Propylenoxid und/oder Butylenoxid-Einheiten durch Styrol-Einheiten substituiert sind.

3. Amidhaltige Polymere nach Anspruch 1 oder 2, wobei im Falle, dass einer oder mehrere der Reste R², R³, R⁴, R⁵, R⁷ und/oder R⁸ einen Polyester-Rest umfassen, dieser Rest oder diese Reste auf der Basis von ein oder mehreren (C₁-C₁₈)-Hydroxycarbonsäuren oder ein oder mehreren Lactonen aufgebaut sind.

4. Amidhaltige Polymere nach Anspruch 3, wobei das Lacton oder die Lactone aus der Gruppe umfassend β-Propiolacton, δ-Valerolacton, ε-Caprolacton und (C₁-C₆)Alkylsubstituiertem ε-Caprolacton, gewählt sind.

5. Amidhaltige Polymere nach einem oder mehreren der Ansprüche 1 bis 4, wobei
R¹ für H, (CH₂)₂-CO-X-R⁵ und/oder CONH-R' mit R' = R⁸ oder -C₆H₃(CH₃)-NHCOO-R⁸ steht,
R² und R⁴ unabhängig voneinander für einen (C₂-C₁₈)Alkylen-, (C₇-C₁₅)Aralkylen-Rest stehen,
R³ für einen (C₂-C₄₀)Alkylen-, (C₃-C₄₀)Alkenylen-, (C₅-C₄₀)Cycloalkylen-, Arylen-, (C₇-C₄₀)Aralkylen-Rest steht,
R⁵ für H, einen (C₁-C₂₂)Alkyl-, (C₅-C₁₂)Cycloalkyl-, Hydroxyalkyl-Rest, einen Hydroxy-, (C₁-C₂₂)Alkoxy-, (C₅-C₁₂)Cycloalkoxy-, oder (C₇-C₁₂)Aralkoxypolyoxyalkylen-Rest, oder einen (C₁-C₂₂)Alkanol-, (C₅-C₁₂)Cycloalkanol-, (C₇-C₁₂)Aralkanol-gestarteten oder einen (C₁-C₂₂)Alkoxy-, (C₅-C₁₂)Cycloalkoxy-, oder (C₇-C₁₂)Aralkoxypolyoxyalkylengestarteten Polyester steht,
R⁶ für einen (C₁-C₃₀)Alkyl- oder einen (C₃-C₂₂)Alkenyl-Rest steht,
R⁷ für einen (C₁-C₁₈)Alkylen- oder einen Polyoxyalkylen-Rest steht,
R⁸ für einen (C₁-C₂₂)Alkyl-Rest steht,
X für gleiche oder verschiedene Reste O oder NH steht,
Y für eine oder mehrere der folgenden Gruppen NHCO und CONH steht und
a und b unabhängig voneinander für eine Zahl von 1 bis 19 stehen.

6. Amidhaltige Polymere nach einem oder mehreren der Ansprüche 1 bis 5, wobei
R¹ für H, (CH₂)₂-CO-X-R⁵ und/oder CONH-R' mit R' = R⁸ oder -C₆H₃(CH₃)-NHCOO-R⁸ steht,
R² und R⁴ unabhängig voneinander für einen (C₂-C₁₂)Alkylen-, (C₇-C₁₂)Aralkylen-Rest stehen,
R³ für einen (C₂-C₄₀)Alkylen-, (C₃-C₄₀)Alkenylen-, (C₅-C₄₀)Cycloalkylen-, Arylen-, (C₇-C₄₀)Aralkylen-Rest steht,
R⁵ für H, einen (C₁-C₂₂)Alkyl-, (C₅-C₁₂)Cycloalkyl-, Hydroxyalkyl-Rest, einen Hydroxy-, (C₁-C₂₂)Alkoxy-, (C₅-C₁₂)Cycloalkoxy-, oder (C₇-C₁₂)Aralkoxypolyoxyalkylen-Rest, oder einen (C₁-C₂₂)Alkanol-, (C₅-C₁₂)Cycloalkanol-, (C₇-C₁₂)Aralkanolgestarteten oder einen (C₁-C₂₂)Alkoxy-, (C₅-C₁₂)Cycloalkoxy-, oder (C₇-C₁₂)Aralkoxypolyoxyalkylen-gestarteten Polyester steht,
R⁶ für einen (C₁₂-C₃₀)Alkyl- oder einen (C₁₂-C₂₂)Alkenyl-Rest steht,
R⁷ für einen (C₁-C₁₈)Alkylen- oder einen Polyoxyalkylen-Rest steht,
R⁸ für einen (C₁-C₂₂)Alkyl-Rest steht,
X für gleiche oder verschiedene Reste O oder NH steht,
Y für eine oder mehrere der folgenden Gruppen NHCO und CONH steht und
a und b unabhängig voneinander für eine Zahl von 1 bis 7 stehen.

7. Amidhaltige Polymere nach einem oder mehreren der Ansprüche 1 bis 6, wobei
R¹ für H, (CH₂)₂-CO-X-R⁵ und/oder CONH-R' mit R' = R⁸ oder -C₆H₃(CH₃)-NHCOO-R⁸ steht,
R² und R⁴ unabhängig voneinander für einen (C₂-C₈)Alkylen-, (C₇-C₉)Aralkylen-Rest stehen,
R³ für einen (C₃₀-C₄₀)Alkylen-, (C₃₀-C₄₀)Alkenylen-, (C₃₀-C₄₀)Cycloalkylen-, Arylen-, (C₃₀-C₄₀)Aralkylen-Rest steht,
R⁵ für H, einen (C₁-C₂₂)Alkyl-, (C₅-C₁₂)Cycloalkyl-, Hydroxyalkyl-Rest, einen Hydroxy-, (C₁-C₂₂)Alkoxy-, (C₅-C₁₂)Cycloalkoxy-, oder (C₇-C₁₂)Aralkoxypolyoxyalkylen-Rest, oder einen (C₁-C₂₂)Alkanol-, (C₅-C₁₂)Cycloalkanol-, (C₇-C₁₂)Aralkanolgestarteten oder einen (C₁-C₂₂)Alkoxy-, (C₅-C₁₂)Cycloalkoxy-, oder (C₇-C₁₂)Aralkoxypolyoxyalkylen-gestarteten Polyester steht,
R⁶ für einen (C₁₂-C₂₀)Alkyl- oder einen (C₁₂-C₂₀)Alkenyl-Rest steht,
R⁷ für einen (C₁-C₁₈)Alkylen- oder einen Polyoxyalkylen-Rest steht,
R⁸ für einen (C₁-C₂₂)Alkyl-Rest steht,
X für gleiche oder verschiedene Reste O oder NH steht,
Y für eine oder mehrere der folgenden Gruppen NHCO und CONH steht und
a und b unabhängig voneinander für eine Zahl von 1 bis 7 stehen.

8. Amidhaltige Polymere gemäß einem oder mehreren der Ansprüche 1 bis 7, **dadurch gekennzeichnet, dass** R¹ für Wasserstoff, CONH-C₁₈H₃₇ und/oder CONH-C₆H₃(CH₃)-NHCOOC₄H₉ steht, und/oder R² und R⁴ für einen Hexamethylen-, Octamethylen- oder m-Xylylen-Rest stehen, und/oder R³ für einen C₃₄-Rest steht, und/oder R⁶ für einen C₁₇-Alkyl- oder einen C₁₇-Alkenyl-Rest steht und/oder X für O oder NH steht und/oder Y für CONH und/oder NHCO steht und/oder a und b jeweils für eine Zahl von 2 bis 5 stehen.

9. Verfahren zur Herstellung amidhaltiger Polymere der allgemeinen Formel (I)
A-X-CO-(CH₂)₂-NR¹-R²-[Y-R³-Y-R⁴]ₐ-B (I)
sowie deren Salze mit Carbonsäuren, Phosphorsäureestern und Sulfonsäuren, wobei
A für R⁵ oder R⁶-Y-[R⁴-Y-R³-Y]_{b}-R²-NR¹-(CH₂)₂-CO-X-R⁷ und
B für Y-R⁶ oder NR¹-(CH₂)₂-CO-X-R⁵ steht, und wobei
R¹ für H, (CH₂)₂-CO-X-R⁵ und/oder CONH-R' mit R' = R⁸ oder -C₆H₃(CH₃)-NHCOO-R⁸ steht,
R², R³, R⁴ und R⁷ unabhängig voneinander für einen (C₁₋C₄₀)Alkylen-, (C₃-C₄₀)Alkenylen-, (C₅-C₄₀)Cycloalkylen-, Arylen-, (C₇-C₄₀)Aralkylen- oder Polyoxyalkylen-Rest oder für einen Polyester-Rest stehen,
R⁵ für H, einen (C₁-C₂₂)Alkyl-, Aryl-, (C₇-C₁₂)Aralkyl-, (C₅-C₁₂)Cycloalkyl-, Hydroxyalkyl-, oder N,N'-Dialkylamino-Rest, einen Hydroxy-, (C₁-C₂₂)Alkoxy-, (C₅-C₁₂)Cycloalkoxy-, oder (C₇-C₁₂)Aralkoxy-Polyoxyalkylen-Rest, oder einen (C₁-C₂₂)Alkanol-, (C₅-C₁₂)Cycloalkanol-, (C₇-C₁₂)Aralkanol-gestarteten oder einen (C₁-C₂₂)Alkoxy-, (C₅-C₁₂)Cycloalkoxy-, oder (C₇-C₁₂)Aralkoxy-Polyoxyalkylen-gestarteten Polyester steht,
R⁶ für einen (C₁-C₃₀)Alkyl-, (C₃-C₂₂)Alkenyl-, Hydroxyalkyl-, (C₄-C₁₃)Cycloalkyl-, Aryl-oder (C₇-C₁₂)Aralkyl-Rest steht,
R⁸ für einen (C₁-C₂₂)Alkyl-, Aryl-, (C₇-C₁₂)Aralkyl-, oder (C₅-C₁₂)Cycloalkylrest, einen (C₁-C₂₂)Alkoxy-, (C₅-C₁₂)Cycloalkoxy-, oder (C₇-C₁₂)Aralkoxy-Polyoxyalkylen-Rest, einen (C₁-C₂₂)Alkanol-, (C₅-C₁₂)Cycloalkanol-, oder (C₇-C₁₂)Aralkanol-gestarteten oder einen (C₁-C₂₂)Alkoxy-, (C₆-C₁₂)Cycloalkoxy-, oder (C₇-C₁₂)Aralkoxy-Polyoxyalkylen-gestarteten Polyester steht,
X für gleiche oder verschiedene Reste O, NH oder NR⁹ steht,
R⁹ für einen (C₁-C₂₂)Alkyl-, Aryl-, (C₇-C₁₂)Aralkyl-, Hydroxyalkyl-, (C₅-C₁₂)CycloalkylRest steht,
Y für eine oder mehrere der folgenden Gruppen COO, OCO, NHCO, CONH, NHCOO, OOCNH, NHCONH steht und
a und b unabhängig voneinander für eine Zahl von 1 bis 19 stehen,
**dadurch gekennzeichnet, dass**,
(A) eine oder mehrere Verbindungen der allgemeinen Formeln (V) und (VIII)
H₂N-R²-[Y-R³-Y-R⁴]ₐ-NH₂ (V)
H₂N-R²-[Y-R³-Y-R⁴]ₐ-YR (VIII)
mit einer oder mehreren Verbindungen der allgemeinen Formeln (VI) und (IX)
R⁵-X-CO-CH=CH₂ (VI)
H₂C=HC-CO-X-R⁷-X-CO-CH=CH₂ (IX)
unter Bildung von Verbindungen mit R¹ = Wasserstoff zur Reaktion gebracht werden, wobei
auf 1 Mol NH₂-Gruppen in den Verbindungen der allgemeinen Formeln (V) und (VIII) 0,8 bis 1,2 Mol CH=CH₂-Gruppen in den Verbindungen der Formeln (VI) und (IX) eingesetzt werden, und
(B) für den Fall, dass R¹ ganz oder teilweise für (CH₂)₂-CO-X-R⁵ und/oder CONH-R' steht, die Verbindungen aus Schritt (A) mit
einer oder mehreren Verbindungen der allgemeinen Formeln (VI) und (VII)
R⁵-X-CO-CH=CH₂ (VI)
R'-NCO (VII)
zur Reaktion gebracht werden, wobei
auf 1 Mol NR¹-Gruppen in den Verbindungen aus Schritt (A) bis zu 1,2 Mol an Verbindungen der allgemeinen Formel (VI) und/oder (VII) eingesetzt werden, und
(C) für den Fall, dass es sich bei den Verbindungen der allgemeinen Formel (I) um Salze von Carbonsäuren, Phosphorsäureestern und Sulfonsäuren handelt, eine Umsetzung der Verbindungen aus Schritt (A) oder (B) mit Carbonsäuren, Phosphorsäureestern und Sulfonsäuren erfolgt.

10. Verfahren zur Herstellung amidhaltiger Polymere nach Anspruch 9, wobei
(A) eine oder mehrere Verbindungen der allgemeinen Formel (V)
H₂N-R₂-[Y-R³-Y-R⁴]ₐ-NH₂ (V)
mit einer oder mehreren Verbindungen der allgemeinen Formel (VI)
R⁵-X-CO-CH=CH₂ (VI)
unter Bildung von Verbindungen mit R¹ = Wasserstoff zur Reaktion gebracht werden, wobei
auf 1 Mol NH₂-Gruppen in den Verbindungen der allgemeinen Formel (V) 0,8 bis 1,2 Mol CH=CH₂-Gruppen in den Verbindungen der Formel (VI) eingesetzt werden, und
(B) für den Fall, dass R¹ ganz oder teilweise für (CH₂)₂-CO-X-R⁵ und/oder CONH-R' steht, die Verbindungen aus Schritt (A) mit
einer oder mehreren Verbindungen der allgemeinen Formeln (VI) und (VII)
R⁵-X-CO-CH=CH₂ (VI)
R'-NCO (VII)
zur Reaktion gebracht werden, wobei
auf 1 Mol NR¹-Gruppen in den Verbindungen aus Schritt (A) bis zu 1,2 Mol an Verbindungen der allgemeinen Formel (VI) und/oder (VII) eingesetzt werden, und
(C) für den Fall, dass es sich bei den Verbindungen der allgemeinen Formel (I) um Salze von Carbonsäuren, Phosphorsäureestern und Sulfonsäuren handelt, eine Umsetzung der Verbindungen aus Schritt (A) oder (B) mit Carbonsäuren, Phosphorsäureestern und Sulfonsäuren erfolgt.

11. Verfahren zur Herstellung amidhaltiger Polymere nach Anspruch 9, wobei
(A) eine oder mehrere Verbindungen der allgemeinen Formel (VIII)
H₂N-R²-[Y-R³-Y-R⁴]ₐ-YR⁶ (VIII)
mit einer oder mehreren Verbindungen der allgemeinen Formel (VI)
R⁵-X-CO-CH=CH₂ (VI)
unter Bildung von Verbindungen mit R¹ = Wasserstoff zur Reaktion gebracht werden, wobei
auf 1 Mol NH₂-Gruppen in den Verbindungen der allgemeinen Formel (VIII) 0,8 bis 1,2 Mol CH=CH₂-Gruppen in den Verbindungen der Formel (VI) eingesetzt werden, und
(B) für den Fall, dass R¹ ganz oder teilweise für (CH₂)₂-CO-X-R⁵ und/oder CONH-R' steht, die Verbindungen aus Schritt (A) mit
einer oder mehreren Verbindungen der allgemeinen Formeln (VI) und (VII)
R⁵-X-CO-CH=CH₂ (VI)
R'-NCO (VII)
zur Reaktion gebracht werden, wobei
auf 1 Mol NR¹-Gruppen in den Verbindungen aus Schritt (A) bis zu 1,2 Mol an Verbindungen der allgemeinen Formel (VI) und/oder (VII) eingesetzt werden, und
(C) für den Fall, dass es sich bei den Verbindungen der allgemeinen Formel (I) um Salze von Carbonsäuren, Phosphorsäureestern und Sulfonsäuren handelt, eine Umsetzung der Verbindungen aus Schritt (A) oder (B) mit Carbonsäuren, Phosphorsäureestern und Sulfonsäuren erfolgt.

12. Verfahren zur Herstellung amidhaltiger Polymere nach Anspruch 9, wobei
(A) eine oder mehrere Verbindungen der allgemeinen Formel (VIII)
H₂N-R²-[Y-R³-Y-R⁴]ₐ-YR⁶ (VIII)
mit einer oder mehreren Verbindungen der allgemeinen Formel (IX)
H₂C=HC-CO-X-R⁷-X-CO-CH=CH₂ (IX)
unter Bildung von Verbindungen mit R¹ = Wasserstoff zur Reaktion gebracht werden, wobei
auf 1 Mol NH₂-Gruppen in den Verbindungen der allgemeinen Formel (VIII) 0,8 bis 1,2 Mol CH=CH₂-Gruppen in den Verbindungen der Formel (IX) eingesetzt werden, und
(B) für den Fall, dass R¹ ganz oder teilweise für (CH₂)₂-CO-X-R⁵ und/oder CONH-R' steht, die Verbindungen aus Schritt (A) mit
einer oder mehreren Verbindungen der allgemeinen Formeln (VI) und (VII)
R⁵-X-CO-CH=CH₂ (VI)
R'-NCO (VII)
zur Reaktion gebracht werden, wobei
auf 1 Mol NR¹-Gruppen in den Verbindungen aus Schritt (A) bis zu 1,2 Mol an Verbindungen der allgemeinen Formel (VI) und/oder (VII) eingesetzt werden, und
(C) für den Fall, dass es sich bei den Verbindungen der allgemeinen Formel (I) um Salze von Carbonsäuren, Phosphorsäureestern und Sulfonsäuren handelt, eine Umsetzung der Verbindungen aus Schritt (A) oder (B) mit Carbonsäuren, Phosphorsäureestern und Sulfonsäuren erfolgt.

13. Verfahren zur Herstellung amidhaltiger Polymere nach einem der Ansprüche 9 bis 11, wobei die in Schritt (B) angegebene Menge der Verbindungen der allgemeinen Formel (VI) nicht in Schritt (B), sondern bereits in Schritt (A) zusätzlich der in Schritt (A) angegebenen Menge der Verbindungen der allgemeinen Formel (VI) eingesetzt wird.

14. Verfahren nach einem oder mehreren der Ansprüche 9 bis 13, wobei die Reaktion in Schritt (A) bei einer Temperatur von 60 bis 100°C, vorzugsweise 70 bis 90 °C durchgeführt wird.

15. Verfahren nach einem oder mehreren der Ansprüche 9 bis 14, wobei die Reaktion in Schritt (B), im Falle der Umsetzung mit Verbindungen der allgemeinen Formel (VII) bei einer Temperatur von 50 bis 100 °C, vorzugsweise 60 bis 80 °C und im Falle der Umsetzung mit einer Verbindung der allgemeinen Formel (VI) bei einer Temperatur von 60 bis 100 °C, vorzugsweise 70 bis 90 °C durchgeführt wird.

16. Verwendung der amidhaltigen Polymere wie in Ansprüchen 1 bis 8 definiert oder erhalten durch das Verfahren nach einem der Ansprüche 9 bis 15, als Rheologiesteuerungsadditiv.

17. Verwendung nach Anspruch 16, wobei das Rheologiesteuerungsadditiv in lösungsmittelfreien und lösungsmittelhaltigen Lacken auf der Basis von Bindemitteln wie Polyurethanen (1K und 2K), Polyacrylaten, Polyester-, Alkyd- und Epoxidharzen, PVC-Plastisolen, und -Organosolen, Beschichtungen auf Epoxidbasis und ungesättigten Polyesterharzen zur Anwendung kommt.

18. Verwendung nach einem der Ansprüche 16 oder 17, wobei das Rheologiesteuerungsadditiv in einer Menge von 0,05 bis 5,0 Gew.-% Wirksubstanz, bevorzugt 0,1 bis 3,0 Gew.-% Wirksubstanz, besonders bevorzugt 0,2 bis 2,0 Gew.-% Wirksubstanz, bezogen auf das Gesamtgewicht der Zusammensetzung in welcher das Rheologiesteuerungsadditiv eingesetzt wird, verwendet wird.

19. Gehärtete und ungehärtete Polymer-Zusammensetzungen enthaltend amidhaltigen Polymere wie in Ansprüchen 1 bis 8 definiert oder wie sie durch das Verfahren nach einem der Ansprüche 9 bis 15 erhalten werden.

## Claims

1. Amide-containing polymers of the general formula (I)
A-X-CO-(CH₂)₂-NR¹-R²-[Y-R³-Y-R⁴]ₐ-B (I)
or their salts with carboxylic acids, esters of phosphoric acid, or sulphonic acids, wherein
A represents R⁵ or R⁶-Y-[R⁴-Y-R³-Y]_{b}-R²-NR¹-(CH₂)₂-CO-X-R⁷ and
B represents Y-R⁶ or NR¹-(CH₂)₂-CO-X-R⁵, and wherein
R¹ represents H, (CH₂)₂-CO-X-R⁵ and/or CONH-R' with R' = R⁸ or -C₆H₃(CH₃)-NHCOO-R⁸,
R², R³, R⁴ and R⁷ independently of one another represent a (C₁-C₄₀)alkylene, (C₃-C₄₀)alkenylene, (C₅-C₄₀)cycloalkylene, arylene, (C₇-C₄₀)aralkylene or polyoxyalkylene group or a polyester group,
R⁵ represents H, a (C₁-C₂₂)alkyl, aryl, (C₇-C₁₂)aralkyl, (C₅-C₁₂)cycloalkyl, hydroxyalkyl, or N,N'-dialkylamino group, a hydroxy, (C₁-C₂₂)alkoxy, (C₅-C₁₂)cycloalkoxy, or (C₇-C₁₂)aralkoxy-polyoxyalkylene group, or a (C₁-C₂₂)alkanol-, (C₅-C₁₂)cycloalkanol-, (C₇-C₁₂)aralkanol-started or a (C₁-C₂₂)alkoxy-, (C₅-C₁₂)cycloalkoxy- or (C₇-C₁₂)aralkoxy-polyoxyalkylene-started polyester,
R⁶ represents a (C₁-C₃₀)alkyl, (C₃-C₂₂)alkenyl, hydroxyalkyl, (C₄-C₁₃)cycloalkyl, aryl-or (C₇-C₁₂)aralkyl group,
R⁸ represents a (C₁-C₂₂)alkyl, aryl, (C₇-C₁₂)aralkyl, or (C₅-C₁₂)cycloalkyl group, a (C₁-C₂₂)alkoxy, (C₅-C₁₂)cycloalkoxy, or (C₇-C₁₂)aralkoxy-polyoxyalkylene group, a (C₁-C₂₂)alkanol-, (C₅-C₁₂)cycloalkanol-, or (C₇-C₁₂)aralkanol-started or a (C₁-C₂₂)alkoxy-, (C₆-C₁₂)cycloalkoxy- or (C₇-C₁₂)aralkoxy-polyoxyalkylene-started polyester,
X represents identical or different groups O, NH or NR⁹,
R⁹ represents a (C₁-C₂₂)alkyl, aryl, (C₇-C₁₂)aralkyl, hydroxyalkyl, (C₅-C₁₂)cycloalkyl group,
Y represents one or more of the following groups COO, OCO, NHCO, CONH, NHCOO, OOCNH, NHCONH and
a and b independently of one another represent a number from 1 to 19.

2. The amide-containing polymers according to claim 1, wherein in the case that one or more of the groups R², R³, R⁴, R⁵, R⁷ and/or R⁸ contain a polyoxyalkylene fraction, this group or these groups is/are constructed from ethylene oxide, propylene oxide and/or butylene oxide units, arranged randomly or in blocks, and optionally one or more of the ethylene oxide, propylene oxide or butylene oxide units are substituted by styrene units.

3. The amide-containing polymers according to claim 1 or 2, wherein in the case that one or more of the groups R², R³, R⁴, R⁵, R⁷ and/or R⁸ contain a polyester group, this group or these groups is/are constructed based on one or more (C₁-C₁₈)-hydroxycarboxylic acids or one or more lactones.

4. The amide-containing polymers according to claim 3, wherein the lactone or the lactones is/are selected from the group comprising β-propiolactone, δ-valerolactone, ε-caprolactone and (C₁-C₆)alkyl-substituted ε-caprolactone.

5. The amide-containing polymers according to one or more of claims 1 to 4, wherein
R¹ represents H, (CH₂)₂-CO-X-R⁵ and/or CONH-R' with R' = R⁸ or -C₆H₃(CH₃)-NHCOO-R⁸,
R² and R⁴ independently of one another represent a (C₂-C₁₈)alkylene, (C₇-C₁₅)aralkylene group,
R³ represents a (C₂-C₄₀)alkylene, (C₃-C₄₀)alkenylene, (C₅-C₄₀)cycloalkylene, arylene, (C₇-C₄₀)aralkylene group,
R⁵ represents H, a (C₁-C₂₂)alkyl, (C₅-C₁₂)cycloalkyl, hydroxyalkyl group, a hydroxy, (C₁-C₂₂)alkoxy-, (C₅-C₁₂)cycloalkoxy, or (C₇-C₁₂)aralkoxy-polyoxyalkylene group, or a (C₁-C₂₂)alkanol-, (C₅-C₁₂)cycloalkanol-, (C₇-C₁₂)aralkanol-started or a (C₁-C₂₂)alkoxy-, (C₅-C₁₂)cycloalkoxy- or (C₇-C₁₂)aralkoxy-polyoxyalkylene-started polyester,
R⁶ represents a (C₁-C₃₀)alkyl or a (C₃-C₂₂)alkenyl group,
R⁷ represents a (C₁-C₁₈)alkylene or a polyoxyalkylene group,
R⁸ represents a (C₁-C₂₂)alkyl group,
X represents identical or different groups O or NH,
Y represents one or more of the following groups NHCO and CONH, and
a and b independently of one another represent a number from 1 to 19.

6. The amide-containing polymers according to one or more of claims 1 to 5, wherein
R¹ represents H, (CH₂)₂-CO-X-R⁵ and/or CONH-R' with R' = R⁸ or -C₆H₃(CH₃)-NHCOO-R⁸,
R² and R⁴ independently of one another represent a (C₂-C₁₂)alkylene, (C₇-C₁₂)aralkylene group,
R³ represents a (C₂-C₄₀)alkylene, (C₃-C₄₀)alkenylene, (C₅-C₄₀)cycloalkylene, arylene, (C₇-C₄₀)aralkylene group,
R⁵ represents H, a (C₁-C₂₂)alkyl, (C₅-C₁₂)cycloalkyl, hydroxyalkyl group, a hydroxy, (C₁-C₂₂)alkoxy-, (C₅-C₁₂)cycloalkoxy, or (C₇-C₁₂)aralkoxy-polyoxyalkylene group, or a (C₁-C₂₂)alkanol-, (C₅-C₁₂)cycloalkanol-, (C₇-C₁₂)aralkanol-started or a (C₁-C₂₂)alkoxy-, (C₅-C₁₂)cycloalkoxy- or (C₇-C₁₂)aralkoxypolyoxyalkylene-started polyester,
R⁶ represents a (C₁₂-C₃₀)alkyl or a (C₁₂-C₂₂)alkenyl group,
R⁷ represents a (C₁-C₁₈)alkylene or a polyoxyalkylene group,
R⁸ represents a (C₁-C₂₂)alkyl group,
X represents identical or different groups O or NH,
Y represents one or more of the following groups NHCO and CONH, and
a and b independently of one another represent a number from 1 to 7.

7. The amide-containing polymers according to one or more of claims 1 to 6, wherein
R¹ represents H, (CH₂)₂-CO-X-R⁵ and/or CONH-R' with R' = R⁸ or -C₆H₃(CH₃)-NHCOO-R⁸,
R² and R⁴ independently of one another represent a (C₂-C₈)alkylene, (C₇-C₉)aralkylene group,
R³ represents a (C₃₀-C₄₀)alkylene, (C₃₀-C₄₀)alkenylene, (C₃₀-C₄₀)cycloalkylene, arylene, (C₃₀-C₄₀)aralkylene group,
R⁵ represents H, a (C₁-C₂₂)alkyl, (C₅-C₁₂)cycloalkyl, hydroxyalkyl group, a hydroxy, (C₁-C₂₂)alkoxy-, (C₅-C₁₂)cycloalkoxy, or (C₇-C₁₂)aralkoxy-polyoxyalkylene group, or a (C₁-C₂₂)alkanol-, (C₅-C₁₂)cycloalkanol-, (C₇-C₁₂)aralkanol-started or a (C₁-C₂₂)alkoxy-, (C₅-C₁₂)cycloalkoxy- or (C₇-C₁₂)aralkoxypolyoxyalkylene-started polyester,
R⁶ represents a (C₁₂-C₂₀)alkyl or a (C₁₂-C₂₀)alkenyl group,
R⁷ represents a (C₁-C₁₈)alkylene or a polyoxyalkylene group,
R⁸ represents a (C₁-C₂₂)alkyl group,
X represents identical or different groups O or NH,
Y represents one or more of the following groups NHCO and CONH, and
a and b independently of one another represent a number from 1 to 7.

8. The amide-containing polymers according to one or more of claims 1 to 7, **characterised in that** R¹ represents hydrogen, CONH-C₁₈H₃₇ and/or CONH-C₆H₃(CH₃)-NHCOOC₄H₉, and/or R² and R⁴ represent a hexamethylene, octamethylene or m-xylylene group, and/or R³ represents a C₃₄ group, and/or R⁶ represents a C₁₇-alkyl or a C₁₇-alkenyl group and/or X represents O or NH and/or Y represents CONH and/or NHCO and/or a and b each represent a number from 2 to 5.

9. A process for the preparation of amide-containing polymers of the general formula (I)
A-X-CO-(CH₂)₂-NR¹-R²-[Y-R³-Y-R⁴]ₐ-B (I)
or their salts with carboxylic acids, esters of phosphoric acid, or sulphonic acids, wherein
A represents R⁵ or R⁶-Y-[R⁴-Y-R³-Y]_{b}-R²-NR¹-(CH₂)₂-CO-X-R⁷ and
B represents Y-R⁶ or NR¹-(CH₂)₂-CO-X-R⁵, and wherein
R¹ represents H, (CH₂)₂-CO-X-R⁵ and/or CONH-R' with R' = R⁸ or -C₆H₃(CH₃)-NHCOO-R⁸,
R², R³, R⁴ and R⁷ independently of one another represent a (C₁-C₄₀)alkylene, (C₃-C₄₀)alkenylene, (C₅-C₄₀)cycloalkylene, arylene, (C₇-C₄₀)aralkylene or polyoxyalkylene group or a polyester group,
R⁵ represents H, a (C₁-C₂₂)alkyl, aryl, (C₇-C₁₂)aralkyl, (C₅-C₁₂)cycloalkyl, hydroxyalkyl, or N,N'-dialkylamino group, a hydroxy, (C₁-C₂₂)alkoxy, (C₅-C₁₂)cycloalkoxy, or (C₇-C₁₂)aralkoxy-polyoxyalkylene group, or a (C₁-C₂₂)alkanol-, (C₅-C₁₂)cycloalkanol-, (C₇-C₁₂)aralkanol-started or a (C₁-C₂₂)alkoxy-, (C₅-C₁₂)cycloalkoxy- or (C₇-C₁₂)aralkoxy-polyoxyalkylene-started polyester,
R⁶ represents a (C₁-C₃₀)alkyl, (C₃-C₂₂)alkenyl, hydroxyalkyl, (C₄-C₁₃)cycloalkyl, aryl-or (C₇-C₁₂)aralkyl group,
R⁸ represents a (C₁-C₂₂)alkyl, aryl, (C₇-C₁₂)aralkyl, or (C₅-C₁₂)cycloalkyl group, a (C₁-C₂₂)alkoxy, (C₅-C₁₂)cycloalkoxy, or (C₇-C₁₂)aralkoxy-polyoxyalkylene group, a (C₁-C₂₂)alkanol-, (C₅-C₁₂)cycloalkanol-, or (C₇-C₁₂)aralkanol-started or a (C₁-C₂₂)alkoxy-, (C₆-C₁₂)cycloalkoxy- or (C₇-C₁₂)aralkoxy-polyoxyalkylene-started polyester,
X represents identical or different groups O, NH or NR⁹,
R⁹ represents a (C₁-C₂₂)alkyl, aryl, (C₇-C₁₂)aralkyl, hydroxyalkyl, (C₅-C₁₂)cycloalkyl group,
Y represents one or more of the following groups COO, OCO, NHCO, CONH, NHCOO, OOCNH, NHCONH, and
a and b independently of one another represent a number from 1 to 19,
**characterised in that**
(A) one or more compounds of the general formulae (V) and (VIII)
H₂N-R²-[Y-R³-Y-R⁴]ₐ-NH₂ (V)
H₂N-R²-[Y-R³-Y-R⁴]ₐ-YR⁶ (VIII)
are brought to reaction with one or more compounds of the general formulae (VI) and (IX)
R⁵-X-CO-CH=CH₂ (VI)
H₂C=HC-CO-X-R⁷-X-CO-CH=CH₂ (IX)
to form compounds with R¹ = hydrogen, wherein
0.8 to 1.2 mol CH=CH₂ groups in the compounds of the formulae (VI) and (IX) are used per 1 mol NH₂ groups in the compounds of the general formulae (V) and (VIII), and
(B) in the case that R¹ in whole or in part represents (CH₂)₂-CO-X-R⁵ and/or CONH-R', the compounds from step (A) are brought to reaction with
one or more compounds of the general formulae (VI) and (VII)
R⁵-X-CO-CH=CH₂ (VI)
R'-NCO (VII),
wherein
up to 1.2 mol of compounds of the general formula (VI) and/or (VII) are used per 1 mol NR¹ groups in the compounds from step (A), and
(C) in the case that the compounds of the general formula (I) are salts of carboxylic acids, of esters of phosphoric acid, or of sulphonic acids, the compounds from step (A) or (B) are reacted with carboxylic acids, esters of phosphoric acid, or sulphonic acids.

10. The process for the preparation of amide-containing polymers according to claim 9, wherein
(A) one or more compounds of the general formula (V)
H₂N-R²-[Y-R³-Y-R⁴]ₐ-NH₂ (V)
are brought to reaction with one or more compounds of the general formula (VI)
R⁵-X-CO-CH=CH₂ (VI)
to form compounds with R¹ = hydrogen, wherein
0.8 to 1.2 mol CH=CH₂ groups in the compounds of formula (VI) are used per 1 mol NH₂ groups in the compounds of the general formula (V), and
(B) in the case that R¹ in whole or in part represents (CH₂)₂-CO-X-R⁵ and/or CONH-R', the compounds from step (A) are brought to reaction with
one or more compounds of the general formulae (VI) and (VII)
R⁵-X-CO-CH=CH₂ (VI)
R'-NCO (VII),
wherein
up to 1.2 mol of compounds of the general formula (VI) and/or (VII) are used per 1 mol NR¹ groups in the compounds from step (A), and
(C) in the case that the compounds of the general formula (I) are salts of carboxylic acids, of esters of phosphoric acid, or of sulphonic acids, the compounds from step (A) or (B) are reacted with carboxylic acids, esters of phosphoric acid, or sulphonic acids.

11. The process for the preparation of amide-containing polymers according to claim 9, wherein
(A) one or more compounds of the general formula (VIII)
H₂N-R²-[Y-R³-Y-R⁴]ₐ-YR⁶ (VIII)
are brought to reaction with one or more compounds of the general formula (VI)
R⁵-X-CO-CH=CH₂ (VI)
to form compounds with R¹ = hydrogen, wherein
0.8 to 1.2 mol CH=CH₂ groups in the compounds of formula (VI) are used per 1 mol NH₂ groups in the compounds of the general formula (VIII), and
(B) in the case that R¹ in whole or in part represents (CH₂)₂-CO-X-R⁵ and/or CONH-R', the compounds from step (A) are brought to reaction with
one or more compounds of the general formulae (VI) and (VII)
R⁵-X-CO-CH=CH₂ (VI)
R'-NCO (VII),
wherein
up to 1.2 mol of compounds of the general formula (VI) and/or (VII) are used per 1 mol NR¹ groups in the compounds from step (A), and
(C) in the case that the compounds of the general formula (I) are salts of carboxylic acids, of esters of phosphoric acid, or of sulphonic acids, the compounds from step (A) or (B) are reacted with carboxylic acids, esters of phosphoric acid, or sulphonic acids.

12. The process for the preparation of amide-containing polymers according to claim 9, wherein
(A) one or more compounds of the general formula (VIII)
H₂N-R²-[Y-R³-Y-R⁴]ₐ-YR⁶ (VIII)
are brought to reaction with one or more compounds of the general formula (IX)
H₂C=HC-CO-X-R⁷-X-CO-CH=CH₂ (IX)
to form compounds with R¹ = hydrogen, wherein
0.8 to 1.2 mol CH=CH₂ groups in the compounds of formula (IX) are used per 1 mol NH₂ groups in the compounds of the general formula (VIII), and
(B) in the case that R¹ in whole or in part represents (CH₂)₂-CO-X-R⁵ and/or CONH-R', the compounds from step (A) are brought to reaction with
one or more compounds of the general formulae (VI) and (VII)
R⁵-X-CO-CH=CH₂ (VI)
R'-NCO (VII),
wherein
up to 1.2 mol of compounds of the general formula (VI) and/or (VII) are used per 1 mol NR¹ groups in the compounds from step (A), and
(C) in the case that the compounds of the general formula (I) are salts of carboxylic acids, of esters of phosphoric acid, or of sulphonic acids, the compounds from step (A) or (B) are reacted with carboxylic acids, esters of phosphoric acid, or sulphonic acids.

13. The process for the preparation of amide-containing polymers according to any one of claims 9 to 11, wherein the amount of compounds of the general formula (VI) indicated in step (B) is not used in step (B), but already in step (A), in addition to the amount of compounds of the general formula (VI) indicated in step (A).

14. The process according to one or more of claims 9 to 13, wherein the reaction in step (A) is effected at a temperature from 60 to 100 °C, preferably 70 to 90 °C.

15. The process according to one or more of claims 9 to 14, wherein the reaction in step (B) is effected at a temperature from 50 to 100 °C, preferably 60 to 80 °C, in the case of reaction with compounds of the general formula (VII), and at a temperature from 60 to 100 °C, preferably 70 to 90 °C, in the case of reaction with a compound of the general formula (VI).

16. Use of the amide-containing polymers as defined in claims 1 to 8, or as obtained by the process according to any one of claims 9 to 15, as a rheology control additive.

17. The use according to claim 16, wherein the rheology control additive is put to use in solvent-free or solvent-containing lacquers on the basis of binders such as polyurethanes (1K and 2K), polyacrylates, polyester-, alkyd- and epoxy resins, PVC plastisols and organosols, epoxy-based coatings and unsaturated polyester resins.

18. The use according to any one of claims 16 or 17, wherein the rheology control additive is used in an amount of 0.05 to 5.0 % by weight of active substance, preferably 0.1 to 3.0 % by weight of active substance, particularly preferably 0.2 to 2.0 % by weight of active substance, based on the total weight of the composition in which the rheology control additive is used.

19. Cured and uncured polymer compositions comprising amide-containing polymers as defined in claims 1 to 8 or as obtained by the process according to one of claims 9 to 15.

## Revendications

1. Polymères contenant des groupes amides de formule générale (I)
A-X-CO-(CH₂)₂-NR¹-R²-[Y-R³-Y-R⁴]ₐ-B (I)
et leurs sels avec des acides carboxyliques, esters d'acide phosphorique et acides sulfoniques, dans lesquels
A représente R⁵ ou R⁶-Y-[R⁴-Y-R³-Y]_{b}-R²-NR¹-(CH₂)₂-CO-X-R⁷ et
B représente Y-R⁶ ou NR¹-(CH₂)₂-CO-X-R⁵, et dans lesquels
R¹ représente H, (CH₂)₂-CO-X-R⁵ et/ou CONH-R' où R' = R⁸ ou -C₆H₃(CH₃)-NHCOO-R⁸,
R², R³, R⁴ et R⁷ représentent, indépendamment les uns des autres, un groupe fonctionnel alkylène en C₁-C₄₀, alcénylène en C₃-C₄₀, cycloalkylène en C₅-C₄₀, arylène, aralkylène en C₇-C₄₀ ou polyoxyalkylène ou un groupe fonctionnel polyester,
R⁵ représente H, un groupe fonctionnel alkyle en C₁-C₂₂, aryle, aralkyle en C₇-C₁₂, cycloalkyle en C₅-C₁₂, hydroxyalkyle ou N,N'-dialkylamino, un groupe fonctionnel hydroxy, alcoxy en C₁-C₂₂, cycloalcoxy en C₅-C₁₂ ou aralcoxy-polyoxyalkylène en C₇-C₁₂, ou un polyester initié par un alcanol en C₁-C₂₂, un cycloalcanol en C₅-C₁₂, un aralcanol en C₇-C₁₂ ou un polyester initié par un alcoxy en C₁-C₂₂, un cycloalcoxy en C₅-C₁₂ ou un aralcoxy-polyoxyalkylène en C₇-C₁₂,
R⁶ représente un groupe fonctionnel alkyle en C₁-C₃₀, alcényle en C₃-C₂₂, hydroxyalkyle, cycloalkyle en C₄-C₁₃, aryle ou aralkyle en C₇-C₁₂,
R⁸ représente un groupe fonctionnel alkyle en C₁-C₂₂, aryle, aralkyle en C₇-C₁₂ ou cycloalkyle en C₅-C₁₂, un groupe fonctionnel alcoxy en C₁-C₂₂, cycloalcoxy en C₅-C₁₂ ou aralcoxy-polyoxyalkylène en C₇-C₁₂, un polyester initié par un alcanol en C₁-C₂₂, un cycloalcanol en C₅-C₁₂, un aralcanol en C₇-C₁₂ ou un polyester initié par un alcoxy en C₁-C₂₂, un cycloalcoxy en C₆-C₁₂ ou un aralcoxy-polyoxyalkylène en C₇-C₁₂,
X représente des groupes fonctionnels identiques ou différents O, NH ou NR⁹,
R⁹ représente un groupe fonctionnel alkyle en C₁-C₂₂, aryle, aralkyle en C₇-C₁₂, hydroxyalkyle, cycloalkyle en C₅-C₁₂,
Y représente un ou plusieurs des groupes fonctionnels suivants COO, OCO, NHCO, CONH, NHCOO, OOCNH, NHCONH et
a et b représentent, indépendamment l'un de l'autre, un nombre compris entre 1 et 19.

2. Polymères contenant des groupes amides selon la revendication 1, dans lesquels, si un ou plusieurs des groupes fonctionnels R², R³, R⁴, R⁵, R⁷ et/ou R⁸ contiennent une fraction de polyoxyalkylène, ce ou ces groupes fonctionnels sont formés à partir d'unités d'oxyde d'éthylène, d'oxyde de propylène et/ou d'oxyde de butylène, agencées de façon statistique ou en blocs, et, le cas échéant, une ou plusieurs des unités d'oxyde d'éthylène, d'oxyde de propylène et/ou d'oxyde de butylène sont substituées par des unités de styrène.

3. Polymères contenant des groupes amides selon la revendication 1 ou 2, dans lesquels, si un ou plusieurs des groupes fonctionnels R², R³, R⁴, R⁵, R⁷ et/ou R⁸ comprennent un groupe fonctionnel polyester, ce ou ces groupes fonctionnels sont formés à partir d'un ou de plusieurs acides hydroxycarboxyliques en C₁-C₁₈ ou d'une ou de plusieurs lactones.

4. Polymères contenant des groupes amides selon la revendication 3, dans lesquels la ou les lactones sont sélectionnées dans le groupe comprenant β-propiolactone, δ-valérolactone, ε-caprolactone et ε-caprolactone substituée par alkyle en C₁-C₆.

5. Polymères contenant des groupes amides selon une ou plusieurs des revendications 1 à 4, dans lesquels
R¹ représente H, (CH₂)₂-CO-X-R⁵ et/ou CONH-R' où R' = R⁸ ou -C₆H₃(CH₃)-NHCOO-R⁸,
R² et R⁴ représentent, indépendamment l'un de l'autre, un groupe fonctionnel alkylène en C₂-C₁₈, aralkylène en C₇-C₁₅,
R³ représente un groupe fonctionnel alkylène en C₂-C₄₀, alcénylène en C₃-C₄₀, cycloalkylène en C₅-C₄₀, arylène, aralkylène en C₇-C₄₀,
R⁵ représente H, un groupe fonctionnel alkyle en C₁-C₂₂, cycloalkyle en C₅-C₁₂, hydroxyalkyle, un groupe fonctionnel hydroxy, alcoxy en C₁-C₂₂, cycloalcoxy en C₅-C₁₂, ou aralcoxypolyoxyalkylène en C₇-C₁₂, ou un polyester initié par un alcanol en C₁-C₂₂, un cycloalcanol en C₅-C₁₂, un aralcanol en C₇-C₁₂ ou un polyester initié par un alcoxy en C₁-C₂₂, un cycloalcoxy en C₅-C₁₂ ou un aralcoxypolyoxyalkylène en C₇-C₁₂,
R⁶ représente un groupe fonctionnel alkyle en C₁-C₃₀ ou alcényle en C₃-C₂₂,
R⁷ représente un groupe fonctionnel alkylène en C₁-C₁₈ ou un groupe fonctionnel polyoxyalkylène,
R⁸ représente un groupe fonctionnel alkyle en C₁-C₂₂,
X représente des groupes fonctionnels identiques ou différents O ou NH,
Y représente un ou plusieurs des groupes fonctionnels suivants NHCO et CONH, et
a et b représentent, indépendamment l'un de l'autre, un nombre compris entre 1 et 19.

6. Polymères contenant des groupes amides selon une ou plusieurs des revendications 1 à 5, dans lesquels
R¹ représente H, (CH₂)₂-CO-X-R⁵ et/ou CONH-R' où R' = R⁸ ou -C₆H₃(CH₃)-NHCOO-R⁸,
R² et R⁴ représentent, indépendamment l'un de l'autre, un groupe fonctionnel alkylène en C₂-C₁₂, aralkylène en C₇-C₁₂,
R³ représente un groupe fonctionnel alkylène en C₂-C₄₀, alcénylène en C₃-C₄₀, cycloalkylène en C₅-C₄₀, arylène, aralkylène en C₇-C₄₀,
R⁵ représente H, un groupe fonctionnel alkyle en C₁-C₂₂, cycloalkyle en C₅-C₁₂, hydroxyalkyle, un groupe fonctionnel hydroxy, alcoxy en C₁-C₂₂, cycloalcoxy en C₅-C₁₂ ou aralcoxypolyoxyalkylène en C₇-C₁₂, ou un polyester initié par un alcanol en C₁-C₂₂, un cycloalcanol en C₅-C₁₂, un aralcanol en C₇-C₁₂ ou un polyester initié par un alcoxy en C₁-C₂₂, un cycloalcoxy en C₅-C₁₂ ou un aralcoxypolyoxyalkylène en C₇-C₁₂,
R⁶ représente un groupe fonctionnel alkyle en C₁₂-C₃₀ ou alcényle en C₁₂-C₂₂,
R⁷ représente un groupe fonctionnel alkylène en C₁-C₁₈ ou un groupe fonctionnel polyoxyalkylène,
R⁸ représente un groupe fonctionnel alkyle en C₁-C₂₂,
X représente des groupes fonctionnels identiques ou différents O ou NH,
Y représente un ou plusieurs des groupes fonctionnels suivants NHCO et CONH, et
a et b représentent, indépendamment l'un de l'autre, un nombre compris entre 1 et 7.

7. Polymères contenant des groupes amides selon une ou plusieurs des revendications 1 à 6, dans lesquels
R¹ représente H, (CH₂)₂-CO-X-R⁵ et/ou CONH-R' où R' = R⁸ ou -C₆H₃(CH₃)-NHCOO-R⁸,
R² et R⁴ représentent, indépendamment l'un de l'autre, un groupe fonctionnel alkylène en C₂-C₈, aralkylène en C₇-C₉,
R³ représente un groupe fonctionnel alkylène en C₃₀-C₄₀, alcénylène en C₃₀-C₄₀, cycloalkylène en C₃₀-C₄₀, arylène, aralkylène en C₃₀-C₄₀,
R⁵ représente H, un groupe fonctionnel alkyle en C₁-C₂₂, cycloalkyle en C₅-C₁₂, hydroxyalkyle, un groupe fonctionnel hydroxy, alcoxy en C₁-C₂₂, cycloalcoxy en C₅-C₁₂, ou aralcoxypolyoxyalkylène en C₇-C₁₂, ou un polyester initié par un alcanol en C₁-C₂₂, un cycloalcanol en C₅-C₁₂, un aralcanol en C₇-C₁₂ ou un polyester initié par un alcoxy en C₁-C₂₂, un cycloalcoxy en C₅-C₁₂ ou un aralcoxypolyoxyalkylène en C₇-C₁₂,
R⁶ représente un groupe fonctionnel alkyle en C₁₂-C₂₀ ou alcényle en C₁₂-C₂₀,
R⁷ représente un groupe fonctionnel alkylène en C₁-C₁₈ ou un groupe fonctionnel polyoxyalkylène,
R⁸ représente un groupe fonctionnel alkyle en C₁-C₂₂,
X représente des groupes fonctionnels identiques ou différents O ou NH,
Y représente un ou plusieurs des groupes fonctionnels suivants NHCO et CONH, et
a et b représentent, indépendamment l'un de l'autre, un nombre compris entre 1 et 7.

8. Polymères contenant des groupes amides selon une ou plusieurs des revendications 1 à 7, **caractérisés en ce que** R¹ représente de l'hydrogène, CONH-C₁₈H₃₇ et/ou CONH-C₆H₃(CH₃)-NHCOOC₄H₉, et/ou R² et R⁴ représentent un groupe fonctionnel hexaméthylène, octaméthylène ou m-xylylène, et/ou R³ représente un groupe fonctionnel C₃₄, et/ou R⁶ représente un groupe fonctionnel alkyle en C₁₇ ou alcényle en C₁₇ et/ou X représente O ou NH et/ou Y représente CONH et/ou NHCO et/ou a et b représentent chacun un nombre compris entre 2 et 5.

9. Procédé de fabrication de polymères contenant des groupes amides de formule générale (I)
A-X-CO-(CH₂)₂-NR¹-R²-[Y-R³-Y-R⁴]ₐ-B (I)
et leurs sels avec des acides carboxyliques, esters d'acide phosphorique et acides sulfoniques, dans lequel
A représente R⁵ ou R⁶-Y-[R⁴-Y-R³-Y]_{b}-R²-NR¹-(CH₂)₂-CO-X-R⁷ et
B représente Y-R⁶ ou NR¹-(CH₂)₂-CO-X-R⁵, et dans lequel
R¹ représente H, (CH₂)₂-CO-X-R⁵ et/ou CONH-R' où R' = R⁸ ou -C₆H₃(CH₃)-NHCOO-R⁸,
R², R³, R⁴ et R⁷ représentent, indépendamment les uns des autres, un groupe fonctionnel alkylène en C₁-C₄₀, alcénylène en C₃-C₄₀, cycloalkylène en C₅-C₄₀, arylène, aralkylène en C₇-C₄₀ ou polyoxyalkylène ou un groupe fonctionnel polyester,
R⁵ représente H, un groupe fonctionnel alkyle en C₁-C₂₂, aryle, aralkyle en C₇-C₁₂, cycloalkyle en C₅-C₁₂, hydroxyalkyle ou N,N'-dialkylamino, un groupe fonctionnel hydroxy, alcoxy en C₁-C₂₂, cycloalcoxy en C₅-C₁₂ ou aralcoxy-polyoxyalkylène en C₇-C₁₂, ou un polyester initié par un alcanol en C₁-C₂₂, un cycloalcanol en C₅-C₁₂, un aralcanol en C₇-C₁₂ ou un polyester initié par un alcoxy en C₁-C₂₂, un cycloalcoxy en C₅-C₁₂ ou un aralcoxy-polyoxyalkylène en C₇-C₁₂,
R⁶ représente un groupe fonctionnel alkyle en C₁-C₃₀, alcényle en C₃-C₂₂, hydroxyalkyle, cycloalkyle en C₄-C₁₃, aryle ou aralkyle en C₇-C₁₂,
R⁸ représente un groupe fonctionnel alkyle en C₁-C₂₂, aryle, aralkyle en C₇-C₁₂ ou cycloalkyle en C₅-C₁₂, un groupe fonctionnel alcoxy en C₁-C₂₂, cycloalcoxy en C₅-C₁₂, ou aralcoxy-polyoxyalkylène en C₇-C₁₂, un polyester initié par un alcanol en C₁-C₂₂, un cycloalcanol en C₅-C₁₂, un aralcanol en C₇-C₁₂ ou un polyester initié par un alcoxy en C₁-C₂₂, un cycloalcoxy en C₆-C₁₂ ou un aralcoxy-polyoxyalkylène en C₇-C₁₂,
X représente des groupes fonctionnels identiques ou différents O, NH ou NR⁹,
R⁹ représente un groupe fonctionnel alkyle en C₁-C₂₂, aryle, aralkyle en C₇-C₁₂, hydroxyalkyle, cycloalkyle en C₅-C₁₂,
Y représente un ou plusieurs des groupes fonctionnels suivants COO, OCO, NHCO, CONH, NHCOO, OOCNH, NHCONH et
a et b représentent, indépendamment l'un de l'autre, un nombre compris entre 1 et 19,
**caractérisé en ce que**
(A) un ou plusieurs composés de formules générales (V) et (VIII)
H₂N-R²-[Y-R³-Y-R⁴]ₐ-NH₂ (V)
H₂N-R²-[Y-R³-Y-R⁴]ₐ-YR⁶ (VIII)
sont réagis avec un ou plusieurs composés de formules générales (VI) et (IX)
R⁵-X-CO-CH=CH₂ (VI)
H₂C=HC-CO-X-R⁷-X-CO-CH=CH₂ (IX)
pour former des composés où R¹ = hydrogène,
sur 1 mol de groupes fonctionnels NH₂ dans les composés de formules générales (V) et (VIII), 0,8 à 1,2 mol de groupes fonctionnels CH=CH₂ étant utilisés dans les composés de formules (VI) et (IX), et
(B) si R¹ représente, intégralement ou en partie, (CH₂)₂-CO-X-R⁵ et/ou CONH-R', les composés issus de l'étape (A) sont réagis avec
un ou plusieurs composés de formules générales (VI) et (VII)
R⁵-X-CO-CH=CH₂ (VI)
R'-NCO (VII),
sur 1 mol de groupes fonctionnels NR¹ dans les composés issus de l'étape (A), jusqu'à 1,2 mol de composés de formules générales (VI) et/ou (VII) étant utilisés, et
(C) si les composés de formule générale (I) sont des sels d'acides carboxyliques, d'esters d'acide phosphorique et d'acides sulfoniques, les composés issus de l'étape (A) ou (B) sont réagis avec des acides carboxyliques, esters d'acide phosphorique et acides sulfoniques.

10. Procédé de fabrication de polymères contenant des groupes amides selon la revendication 9, dans lequel
(A) un ou plusieurs composés de formule générale (V)
H₂N-R²-[Y-R³-Y-R⁴]ₐ-NH₂ (V)
sont réagis avec un ou plusieurs composés de formule générale (VI)
R⁵-X-CO-CH=CH₂ (VI)
pour former des composés où R¹ = hydrogène,
sur 1 mol de groupes fonctionnels NH₂ dans les composés de formule générale (V), 0,8 à 1,2 mol de groupes fonctionnels CH=CH₂ étant utilisés dans les composés de formule (VI), et
(B) si R¹ représente, intégralement ou en partie, (CH₂)₂-CO-X-R⁵ et/ou CONH-R', les composés issus de l'étape (A) sont réagis avec
un ou plusieurs composés de formules générales (VI) et (VII)
R⁵-X-CO-CH=CH₂ (VI)
R'-NCO (VII),
sur 1 mol de groupes fonctionnels NR¹ dans les composés issus de l'étape (A), jusqu'à 1,2 mol de composés de formules générales (VI) et/ou (VII) étant utilisés, et
(C) si les composés de formule générale (I) sont des sels d'acides carboxyliques, d'esters d'acide phosphorique et d'acides sulfoniques, les composés issus de l'étape (A) ou (B) sont réagis avec des acides carboxyliques, esters d'acide phosphorique et acides sulfoniques.

11. Procédé de fabrication de polymères contenant des groupes amides selon la revendication 9, dans lequel
(A) un ou plusieurs composés de formule générale (VIII)
H₂N-R²-[Y-R³-Y-R⁴]ₐ-YR⁶ (VIII)
sont réagis avec un ou plusieurs composés de formule générale (VI)
R⁵-X-CO-CH=CH₂ (VI)
pour former des composés où R¹ = hydrogène,
sur 1 mol de groupes fonctionnels NH₂ dans les composés de formule générale (VIII), 0,8 à 1,2 mol de groupes fonctionnels CH=CH₂ étant utilisés dans les composés de formule (VI), et
(B) si R¹ représente, intégralement ou en partie, (CH₂)₂-CO-X-R⁵ et/ou CONH-R', les composés issus de l'étape (A) sont réagis avec
un ou plusieurs composés de formules générales (VI) et (VII)
R⁵-X-CO-CH=CH₂ (VI)
R'-NCO (VII),
sur 1 mol de groupes fonctionnels NR¹ dans les composés issus de l'étape (A), jusqu'à 1,2 mol de composés de formules générales (VI) et/ou (VII) étant utilisés, et
(C) si les composés de formule générale (I) sont des sels d'acides carboxyliques, d'esters d'acide phosphorique et d'acides sulfoniques, les composés issus de l'étape (A) ou (B) sont réagis avec des acides carboxyliques, esters d'acide phosphorique et acides sulfoniques.

12. Procédé de fabrication de polymères contenant des groupes amides selon la revendication 9, dans lequel
(A) un ou plusieurs composés de formule générale (VIII)
H₂N-R²-[Y-R³-Y-R⁴]ₐ-YR⁶ (VIII)
sont réagis avec un ou plusieurs composés de formule générale (IX)
H₂C=HC-CO-X-R⁷-X-CO-CH=CH₂ (IX)
pour former des composés où R¹ = hydrogène,
sur 1 mol de groupes fonctionnels NH₂ dans les composés de formule générale (VIII), 0,8 à 1,2 mol de groupes fonctionnels CH=CH₂ étant utilisés dans les composés de formule (IX), et
(B) si R¹ représente, intégralement ou en partie, (CH₂)₂-CO-X-R⁵ et/ou CONH-R', les composés issus de l'étape (A) sont réagis avec
un ou plusieurs composés de formules générales (VI) et (VII)
R⁵-X-CO-CH=CH₂ (VI)
R'-NCO (VII),
sur 1 mol de groupes fonctionnels NR¹ dans les composés issus de l'étape (A), jusqu'à 1,2 mol de composés de formules générales (VI) et/ou (VII) étant utilisés, et
(C) si les composés de formule générale (I) sont des sels d'acides carboxyliques, d'esters d'acide phosphorique et d'acides sulfoniques, les composés issus de l'étape (A) ou (B) sont réagis avec des acides carboxyliques, esters d'acide phosphorique et acides sulfoniques.

13. Procédé de fabrication de polymères contenant des groupes amides selon l'une des revendications 9 à 11, dans lequel la quantité de composés de formule générale (VI) indiquée à l'étape (B) est utilisée non pas à l'étape (B) mais dès l'étape (A), en sus de la quantité de composés de formule générale (VI) indiquée à l'étape (A).

14. Procédé selon une ou plusieurs des revendications 9 à 13, dans lequel la réaction de l'étape (A) est réalisée à une température de 60 à 100 °C, de préférence 70 à 90 °C.

15. Procédé selon une ou plusieurs des revendications 9 à 14, dans lequel la réaction de l'étape (B), dans le cas de la reaction avec des composés de formule générale (VII), est réalisée à une température de 50 à 100°C, de préférence 60 à 80 °C, et dans le cas de la reaction avec un composé de formule générale (VI), à une température de 60 à 100 °C, de préférence 70 à 90 °C.

16. Utilisation des polymères contenant des groupes amides tels que définis dans les revendications 1 à 8, ou obtenus par le procédé selon l'une des revendications 9 à 15, comme additif de contrôle de la rhéologie.

17. Utilisation selon la revendication 16, dans laquelle l'additif de contrôle de la rhéologie est utilisé dans des vernis sans solvant et avec solvant à base de liants, tels que polyuréthanes (1 K et 2K), polyacrylates, résines polyester, alkyde et époxy, plastisols et organosols PVC, revêtements à base d'époxyde et résines polyester insaturées.

18. Utilisation selon l'une des revendications 16 ou 17, dans laquelle l'additif de contrôle de la rhéologie est utilisé dans une quantité de substance active comprise entre 0,05 et 5,0 % en poids, de préférence 0,1 et 3,0 % en poids, plus préférablement 0,2 et 2,0 % en poids, par rapport au poids total de la composition dans laquelle est utilisé l'additif de contrôle de la rhéologie.

19. Compositions polymères durcies et non durcies contenant des polymères contenant des groupes amides, tels que définis dans les revendications 1 à 8 ou tels qu'obtenus par le procédé selon l'une des revendications 9 à 15.
